(19) Europäisches Patentamt
European Patent Office
Office européen des brevets

(11) EP 1 928 431 B1

(12) **EUROPEAN PATENT SPECIFICATION**

(45) Date of publication and mention of the grant of the patent:
**31.12.2008 Bulletin 2009/01**

(21) Application number: **06779688.8**

(22) Date of filing: **23.08.2006**

(51) Int Cl.:
***A61K 9/50*** *(2006.01)* ***A61K 31/403*** *(2006.01)*

(86) International application number:
**PCT/HU2006/000068**

(87) International publication number:
**WO 2007/023325 (01.03.2007 Gazette 2007/09)**

(54) **CONTROLLED RELEASE PHARMACEUTICAL COMPOSITION CONTAINING CARVEDILOL**

CARVEDILOL ENTHALTENDE PHARMAZEUTISCHE ZUSAMMENSETZUNG MIT KONTROLLIERTER FREISETZUNG

COMPOSITION PHARMACEUTIQUE A LIBERATION LENTE CONTENANT CARVEDILOL

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **26.08.2005 HU 0500803 P**

(43) Date of publication of application:
**11.06.2008 Bulletin 2008/24**

(73) Proprietor: **Egis Gyógyszergyár Nyilvánosan Múködö Részvénytársaság**
**1106 Budapest (HU)**

(72) Inventors:
- **FEKETE, Pál**
**H-1147 Budapest (HU)**
- **BUDAVARI, Zoltán**
**H-1124 Budapest (HU)**
- **ZSIGMOND, Zsolt**
**H-1173 Budapest (HU)**
- **BOZSO, Agnes**
**H-2060 Bicske (HU)**
- **LEVENTISZNE HUSZAR, Magdolna**
**H-1125 Budapest (HU)**
- **PALFI, Zoltánné**
**H-1172 Budapest (HU)**
- **SZENTGROTI, Pálné**
**H-1071 Budapest (HU)**
- **DAVIDNE KARACS, Erika**
**H-2220 Vecsés (HU)**
- **TUROCZINE KUNCZE, Anikó**
**H-1165 Budapest (HU)**
- **ABRAHAM, Krisztina**
**H-2711 Tápioszentmárton (HU)**

(74) Representative: **Beszédes, Stephan G. et al**
**Münchner Strasse 80 A**
**D-85221 Dachau (DE)**

(56) References cited:
**WO-A-03/074032**
**WO-A-20/04056336**
**WO-A2-02/065834**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## FIELD OF THE INVENTION

Carvedilols, 1-[9'H-carbazol-4'-yloxy]-3-[{2"-(2""-<methoxy>-phenoxy)-ethyl}-amino]-propan-2-ol of the formula

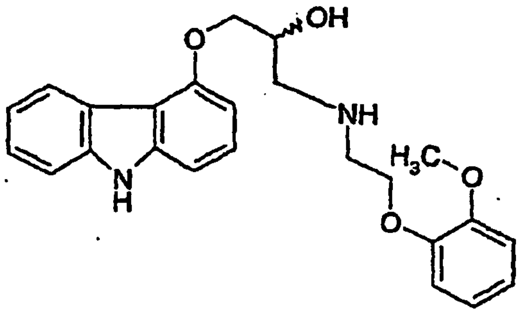

(I)

is a non-selective, $\alpha$ and $\alpha_1$ adrenoreceptor antagonist which is used for the long-term treatment of essential hypertonia and angina pectoris. The $\beta$-adrenoreceptor antagonistic effect of the compound inhibits the development of reflex tachycardia and the $\alpha_1$ adrenoreceptor antagonistic effect causes vasodilatation. Furthermore the compound has antioxidant properties therefore it can be used for the treatment of decompensation as well.

The currently marketed pharmaceutical compositions of carvedilol are immediate release tablets or film coated tablets. The active ingredient dissolves in the stomach and allows the immediate absorption of the active ingredient. The use of these pharmaceutical compositions calls for administration twice a day, and the high plasma concentration caused by the immediate absorption of an active ingredient can cause side effects, such as dinus, headache, fatigue, orthostasis, black-out, dyspnoe, bradychardia, hypertension, etc.

Because of the side effects described above in the case of carvedilol a pharmaceutical composition should be developed, that releases the active ingredient in a period of a few hours, instead of immediate release. These compositions are referred in the literature as "controlled release compositions".

## TECHNICAL BACKGROUND OF THE INVENTION

According to prior art, three different types of controlled-release pharmaceutical compositions are known, such as matrix compositions, coated osmotic compositions or coated diffusion compositions.

In the case of matrix composition the tablets are prepared from the mixture of the active pharmaceutical ingredient and the suitable auxiliary agents from which the active pharmaceutical ingredient dissolves by diffusion or during the disintegration of the matrix. The preparation of these compositions is cheap, but the passing time of these compositions through the gastrointestinal tract is incidental, the composition can be stopped in the stomach or in the different parts of the gastrointestinal tract, therefore the plasma concentrations show in most cases very high deviation in case of compositions having optimal in vitro dissolution (e.g. zero order kinetic, pH independent dissolution) as well.

In case of coated osmotic compositions the tablets are essentially same as the matrix tablets. The tablets are coated with a semipermeable membrane and a hole is drilled through the coating to help the dissolution of the active ingredient. Following, administration the liquid is adsorbed into the tablet through the semipermeable coating, dissolves the active pharmaceutical ingredients and/or auxiliary agents, the resulting osmotic pressure push out the content of the tablet through the holes of the coating. Although these compositions are very suitable to achieve the optimal in vitro dissolution profile, as the disadvantages showed above with reference to matrix tablets can happen in this case of use of the coated osmotic compositions, the plasma concentrations usually also show very high deviation.

The coated diffusion compositions do not contain the active pharmaceutical ingredient in a single portion, but in the form of a large number of small particles, generally coated particles (spheroid granules i.e. pellets) having 0,2-2,0 mm diameter and filled in capsules or pressed to tablets in order to obtain the final pharmaceutical compositions. Since, after the disintegration of the tablet or capsule in the stomach, the coated particles get mixed with the content of the stomach and pass smoothly through the gastrointestinal tract, the advantages of optimal in vitro dissolution can be maximized, and the dissolution of the active ingredient in the different parts of the gastrointestinal tract - each part having different absorption rates - do not result in too high or too low concentrations of the active ingredient, the plasma

concentration and the effects of the pharmaceutical composition become more moderate and the risk of side effects is reduced.

Carvedilol has disadvantageous properties from the point of view of the preparation of controlled release compositions. The weak base carvedilol has low solubility in water depending on the pH of the solution. In the range of pH 1 to 8 solubility decreases from 1 mg/ml to 0.01 mg/ml. Such low solubility of carvedilol in the intestinal tract where the pH is 6.5-7 causes very low absorption. For example, if in case of an orally administered and absorbed active ingredient, the absorbtion rate is 100%, the absorption of the composition administered in the jejunum is 56%, in ileum 28%, and in the colon only 7%. Because of the circumstances above, the preparation of a controlled release pharmaceutical formula requires special technical measures.

There are three frequently used special technics to serve the improved dissolution of carvedilol directly in aqueous media, namely: the preparation of new salts, which have better solubility in water; preparation of complex compounds, and the use of suitable auxiliary agents.

According to the international patent application No. WO 03/092622, carvedilol monocitrate is 100 times more soluble in water than carvedilol base. According to the international patent application No. W02004/002419 the phosphate salt of carvedilol and according to the international patent application No. WO2004/002472 the hydrobromide salt and their solvates can be applied. These compounds have significantly higher solubility in water than the carvedilol base. None of these patent applications contain descriptions of the use of these compounds in controlled release pharmaceutical compositions.

According to international patent application No. WO01/35958 the solubility of carvedilol methansulphonate salt in water is (8 mg/ml) considerably better than that of carvedilol base (0.01 mg/ml); therefore, according to the inventors, this salt is suitable for the preparation of a controlled release composition. Although some tablet and coated tablet compositions are described in the patent application referred to, neither in vitro dissolution data nor in vivo data have been published.

Special cyclodextrine complexes of carvedilol (sulphobutylether-β-cyclodextrin and hydroxypropyl cyclodextrin complexes) are mentioned in the international patent application No. WO 03/028718. The solubility of these compounds is considerably higher than the solubility of free bases. Using of these compounds better drug absorption may be achieved in the colon, therefore the authors suggest the use of these compounds for the preparation of controlled release pharmaceutical compositions. The description does not contain any suggestion for the formulation of solid controlled-release compositions. Although the in vivo data of the described oral suspensions showed extended effect in dog experiments, the deviation of the data is very broad.

On the basis of the facts described above it is ambiguous whether the use of the suggested new salt, solvates and complexes for the preparation of a controlled release pharmaceutical composition is possible or not. Further problem is that these compounds are essentially new chemical compounds and therefore the required chemical, physiological and clinical testing involve more time and higher costs.

Other measures for developing controlled release compositions containing carvedilol with appropriate absorption address special pharmaceutical compositions.

Because of the low solubility of carvedilol in case of matrix compositions the dissolution of the active ingredient does not take part through diffusion but only through the gradual decomposition of the matrix.

The matrix composition according to the international patent application WO99/24017 contains a hydrophilic polymer (hydroxypropylmethylcellulose, polyacrylic acid (Carbopol)), mannitol and an enteric coating layer if necessary.

The matrix (osmotic) composition according to international patent application WO02/092078 contains a hydrophilic polymer (polyethylene oxyde) and microcrystalline cellulose and if necessary an osmotic auxiliary agent and a semipermeable coating with suitable holes.

The matrix composition according to international patent application WO02/065834 contains a physiologically inert organic acid (citric acid), and, if necessary, an enteric coating layer.

The matrix composition according to the international patent application WO2004/016249 contains a swelling cellulose derivative, an anionic polymer (alginic acid derivative) and a cationic polymer (methacrylic acid dimethylaminoethylamino copolymer).

A special matrix composition is disclosed in the description of the international patent application WO 03/024426 in which the cylindriform core of the composition contains a solid dispersion of carvedilol and a hydrophilic polymer and the core is surrounded by a pipe-like, open ended coating (containing ethylcellulose) which is erodable in water.

The use of enteric coating in case of carvedilol according to the international patent applications WO99/24017 and WO02/065834 causes the decrease of the dissolution of carvedilol after the acidic treatment supposedly because of chemical bound formed between carvedilol and the enteric coating polymer used. These effects have been examined in our experiments in which the dissolution of carvedilol was examined from capsules with enteric coating.

As we mentioned above, the disadvantages of matrix compositions are characteristic for all matrix compositions which are unit dose. During the passing of these compositions through the gastrointestinal tract, the composition can be stopped in the stomach or in the different parts of the gastrointestinal tract, therefore the plasma concentrations show

in most cases very high deviation from the optimal values in in vitro dissolution.

According to international patent application WO98/10754, if the usual pellets containing carvedilol and microcrystalline cellulose and of enteric coated pellets, the dissolution of carvedilol takes place only in part because of the very low solubility of carvedilol.

The instructions of the application call for immediately degradable pellets and propose the use of surfactants. These pellets are coated with layers of different thickness therefore become degraded at different times because of the different swelling rate of the core, and release the active ingredient more or less at the required time intervals. The theory is that the desired dissolution profile can be achieved with the appropriate mixture of the pellets having different coating layers. An examinations of these pellets show that the dissolution profiles of coated and uncoated pellets can be very different. However, as the description does not contain any data about the dissolution profiles of the compositions in aqueous media of different pH values, it is not predictable whether the compositions prepared according to the proposed process are really appropriate.

According to international patent application No. WO02/080887 the composition with a chapped coating is prepared in a tablet form instead of the pellet form. The applicability of the pharmaceutical composition in this case is disputable because not all of these tablets work as designed and therefore severe side effects can be caused by the dissolution of a large amount of active ingredient if the tablet hangs up in the stomach.

WO 2004/056336 discloses a multiple unit dosage form, each unit comprising a core, a coating layer including carvedilol, and a rate controlling layer.

Taking into consideration the possible faults of the technical solutions described above, we wished to develop a composition containing particulates which have a uniform dissolution profile in the range of pH 1.2-6.8, and are likely capable to maintain constant plasma concentration.

## SUMMARY OF THE INVENTION

The object of the present invention has been to develop a controlled release pharmaceutical composition in a layered pellet form containing carvedilol which composition has (1) a core containing a solid organic acid, (2) an enteric coating layer on the core, (3) a layer containing carvedilol and a water-soluble binder on the surface of the enteric coating and a dissolution controlling layer containing a mixture of a water-soluble and an enteric polymer shown on Fig.1.

## DETAILED DESCRIPTION OF THE INVENTION

During the development of controlled release composition we found surprisingly that the composition that meets the requirements of therapy can be prepared in a layered pellet form in which the core (1) of the pellet contains a solid organic acid neither toxic nor causing the degradation of carvedilol. This core is covered with an enteric coating (2) which inhibits the dissolution and is coated with the layer containing carvedilol (3) topped with another dissolution controlling layer (4) that contains a mixture of an enteric and a water-soluble polymer. These pellets can be filled in hard gelatine capsules or pressed to tablets to form the final pharmaceutical composition.

The present invention is based on two recognitions. First, it was found very surprisingly that it is not advantageous to use carvedilol and the solid organic acid which helps its dissolution, in the same layer (in the same matrix), because the dissolution of the solid organic acids in aqueous media is quicker, leaving the carvedilol particles slowing down or stopping their dissolution. Therefore, the organic acid has to be placed into a different layer, namely into the core, because it has to restrict the dissolution of the organic acid in the stomach. For achieving this aim, the core has to be provided with a coating, that is insoluble in acidic media but soluble above pH 5.5 the intestinal tract.

The other surprising finding is that the layer, which controls the dissolution of carvedilol has to contain a mixture of a water-soluble film coating polymer and an enteric film coating polymer and it must not contain film coating polymers which do not dissolve in aqueous media and are used according to the prior art. In the case of the use of polymers which do not dissolve in water, the pH independent dissolution of the active ingredient can not be achieved. We found when using enteric film coating polymer alone that the dissolution stops at the dissolution rate of 60-70% of the carvedilol. We found surprisingly that the appropriate dissolution can be achieved by the use of a control layer containing a mixture of water-soluble and enteric film coating polymers.

The term pellet core (core) is used as it is generally used in the pharmaceutical industry. The core is the innermost optionally spheroid part of the pellet, on which some layers of the different compositions are placed.

According to the present invention the solid organic acids in the pellet core, which do not cause the degradation of the carvedilol are saturated, non-saturated di- and tricarboxylic acids containing 4-5 carbon atoms, preferably citric acid, tartaric acid, malic acid, maleic acid, succinic acid. But fumaric acid can not be used due to interaction between carvedilol and fumaric acid.

The present invention defines enteric polymers as polymers, which are able to form a gastric-acid-resistant coating layer soluble in the intestinal tract. Suitable polymers for preparation of enteric layers are for example the

methacrylic acid - methyl methacrylate copolymer, metacrylic acid-ethyl acrylate copolymer, cellulose acetate phtalate, hydroxypropylmethylcellulose phtalate, hydroxypropylmethylcellulose acetate succinate or polyvinylacetate phtalate.

In the present invention the term "water-soluble binding agents" refer to water-soluble polymers which are generally used in the pharmaceutical industry, or to a mixture of these polymers. For example, polymers can be cellulose derivatives - such as hydroxypropylcellulose, hydroxypropylmethylcellulose, hydroxyethylcellulose, or glycol derivatives, as polyethylene glycols and their copolymers, such as polyvinylalcohol polyethylene glycol graft copolymer, or vinylpyrrolidone derivatives such as polyvinylpyrrolidone or vinylpyrrolidon/vinylacetate copolymer or polyvinyl alcohols or their mixtures.

According to the present invention, the dissolution control layer is a layer which covers the layer containing the active ingredient and which has the function of controlling the dissolution of the active ingredient. It contains a mixture of a water-soluble film coating polymer and an enteric film coating polymer. Polymers, which, according to the present invention, can be used for the preparation of the layer containing carvedilol, are able to function as water-soluble polymer and polymers listed above as enteric film coating polymers for the preparation of the enteric film coating on the surface of the core can be used.

More particularly the object of the present invention is to produce a controlled release pharmaceutical composition containing carvedilol in layered pellet form with the core (1) of the pellet containing 5-50 weight%, preferably 10-40 weight%, more preferably 20-40 weight% of solid organic acid based on the total weight of the pellet. Optionally the core contains 5-20 weight%, preferably 10-40 weight%, more preferably 20-40 weight% of one ore more auxiliary agents; an enteric layer (2) on the surface of the core containing 0,5-5 weight%, preferably 1-5 weight%, more preferably 1-3 weight% of enteric polymer based on the total weight of the pellet and optionally further auxiliary agents; on the surface of the enteric layer is a further layer (3) containing 5-30 weight%, preferably 10-20 weight%, more preferably 10-15 weight% of carvedilol based on the total weight of the pellet and 5-30 weight%, preferably 10-20 weight%, more preferably 10-15 weight% of water-soluble binder based on the total weight of the pellet and optionally further auxiliary agents, on the surface of the layer containing carvedilol, a further layer (4) containing a mixture of 0.5-10 weight%, preferably 1-5 weight%, more preferably 1-3 weight% of water-soluble polymer based on the total weight of the pellet and 0.5-10 weight%, preferably 1-5 weight%, more preferably 1-3 weight% of enteric polymer based on the total weight of the pellet and optionally further auxiliary agents.

The pellet core (1) contains saturated or unsaturated organic di- or tricarboxylic acids having 4-5 carbon atoms excluding fumaric acid, preferably citric acid, tartaric acid, malic acid, maleic acid, succinic acid - more preferably succinic acid.

Optionally the pellet core (1) can contain one or more auxiliary agents used generally in the pharmaceutical industry for the preparation of pellets, preferably lactose, starch, powdered cellulose, microcrystalline cellulose, colloid silicone dioxide and dimethylpolysiloxane or their mixtures, more preferably microcrystalline cellulose and dimethylpolysiloxane.

Enteric polymers, preferably methacrylic acid - methyl methacrylate copolymer, methacrylic acid-ethyl acrylate copolymers, cellulose acetate phtalate, hydroxypropylmethylcellulose phtalate, hydroxypropylmethylcellulose acetate succinate or polyvinylacetate phtalate can be used as enteric layer (2) on the surface of the core (1). The enteric layer (2) optionally can contain softeners as other auxiliary agents and antiadhesive agents too. In this case propyleneglycol, triethylcitrate, polyethyleneglycol or their mixtures can be used as softeners, talc, dimethylpolysiloxane or their mixtures can be used as antiadhesive agents.

The layer containing carvedilol (3) can also contain hydroxypropylcellulose, hydroxypropylmethylcellulose, hydroxyethylcellulose, polyvinylpyrrolidone, vinylpyrrolidon/vinylacetate copolymer polyvinyl alcohol, polyethylenglycol, polyvinylalcohol-polyethylenglycol copolymer, or their mixtures as water-soluble binder.

The dissolution control layer (4) on the surface of the layer with carvedilol (3) contains hydroxypropylcellulose, hydroxypropylmethylcellulose, hydroxyethylcellulose, polyvinylpyrrolidone, vinylpyrrolidon/vinylacetate copolymer, polyvinyl alcohol, polyethylenglycol, polyvinylalcohol-polyethylenglycol copolymer or their mixtures, preferably hydroxypropylcellulose as water-soluble polymer and methacrylic acid - methyl methacrylate copolymer, methacrylic acid-ethyl acrylate copolymer, cellulose acetate phtalate, hydroxypropylmethylcellulose phtalate, hydroxypropylmethylcellulose acetate succinate or polyvinylacetate phtalate or their mixtures, preferably methacrylic acid-ethyl acrylate copolymer as enteric polymer.

The preferable embodiment of the present invention as a controlled release pharmaceutical composition containing carvedilol in layered pellet form which contains a core (1) of the pellet containing 25-35 weight% of **succinic** acid, 25-35 weight% of microcrystalline cellulose and 0.4-1 weight% of dimethylpolysiloxane based on the total weight of the pellet, an enteric layer (2) on the surface of the core containing 1-5 weight% methacrylic acid-ethyl acrylate copolymer and optionally 0.1-0.35 weight% of propylene glycol based on the total weight of the pellet; and there is on the surface of the enteric layer a further layer (3) containing 10-15 weight%, of carvedilol based on the total weight of the pellet and 10-15 weight% of a mixture of hydroxypropylmethylcellulose and polyethylene glycol based on the total weight of the pellet and optionally further auxiliary agents, on the surface of the layer containing carvedilol a further layer

(4) containing a mixture of 1-3 weight% of hydroxypropylcellulose based on the total weight of the pellet and 1-3 weight%, of methacrylic acid - ethylacrylate copolymer based on the total weight of the pellet. There may also be further auxiliary agents.

Further object of the present invention is a process for the preparation of a controlled release pharmaceutical composition containing carvedilol in layered pellet form in the following way:

a.) A solid organic acid or one or more auxiliary agents are granulated in a known manner and the granules are dried if it is necessary;

b.) The obtained pellet cores (1) are coated with an enteric layer (2) containing an enteric film coating polymer and optionally one or more auxiliary agents. If it is necessary the obtained coated pellets are dried.

c.) The pellets are coated in a known manner with a layer (3) containing a mixture of the active ingredient and a water-soluble binding agent and optionally one or more auxiliary agents. If it is necessary the obtained coated pellets are dried,

d.) The obtained layer (3) containing carvedilol is coated in a known manner with a layer (4) containing a mixture of a water-soluble film coating polymer and an enteric film coating polymer.

The preparation of coated pellets is known to persons skilled in the art, for the process is accomplished with apparatus and materials generally applied for similar purposes in the pharmaceutical industry. The core can also be prepared using different processes, for instance build-up granulation, centrifugal granulation, extrusion-spheroaisation, high-shear granulation and fluidisation rotogranulation processes.

The enteric layer (2) which is on the pellet core and controls the dissolution of the solid organic acid compound from the core, the layer containing the active ingredient (3) and the layer (4) which controls the dissolution of the active ingredient, can be prepared also by build-up granulation, centrifugal granulation, Wurster fluidisation, fluidisation rotogranulation process.

The optimisation of the technological parameters depending on the process and apparatus used is the duty of the person skilled in the art. The polymers can be used as organic solutions or aqueous dispersions depending on the apparatus available or the quality of the polymers in question.

The enteric layer besides the enteric coating polymer may contain further auxiliary agents, including softeners e.g. propylene glycol, triethylcitrate, polyethylene glycol and so on. It can also comprise antiadhesive agents e.g. talc, dimethylpolysiloxane or other auxiliary agents.

The layer containing the active ingredient (3) consists of a mixture of carvedilol and a water-soluble binding agent, and may also include further auxiliary agents. Every water-soluble binding agent generally used in the pharmaceutical industry for the similar purposes can be used as a water-soluble binding agent. Such agents are for example polyvinylpyrrolidone, vinylpyrrolidon/vinylacetate copolymer, polyvinylalcohol, polyethylene glycol, polyvinylalcohol-polyethylene glycol graft copolymer, hydroxypropylcellulose hydroxyethylcellulose, hydroxypropylmethylcellulose and especially a mixture of hydroxymethylcellulose and polyethylene glycol. The layer may also comprise further auxiliary agents, especially dimethylpolysiloxane, which has antiadhesive property.

The layer containing the active ingredient can be prepared with processes known by those skilled in the art, for example, the build-up granulation, centrifugal granulation, Wurster fluidisation, fluidisation rotogranulation process. The active ingredient can be introduced into the pellet by the subsequent application of the aqueous solution of the water-soluble binding agent and the powder of the active ingredient, or by the application a suspension of the powder and the aqueous solution of the binding agent

In the present invention, the external layer (4) which controls the dissolution of the active ingredient, contains a mixture of enteric film-coating polymer and a water-soluble film-coating polymer. As water-soluble polymers, the same polymers should be suitable that are mentioned above in the description of the layer containing the active ingredient (3). As enteric polymers, the same polymers can be used which are listed at the description of the enteric coating layer (2) on the core. Especially advantageous is the use of a mixture of hydroxypropylcellulose and methacrylic acid-ethylacrylate copolymer from the point of view of industrial application, and the dissolution profile of the active ingredient. The polymers can be used either as a solution in an organic solvent or as an aqueous dispersion. Besides, the coating the polymers can contain further accessory agents, softeners e.g. propylene glycol, triethylcitrate, polyethylene glycol, and so on, and antiadhesive agents like talc or dimethylpolysiloxane.

The layer controlling the dissolution of the active ingredient can be prepared with an arbitrary process known by the skilled in the art. For example, the build-up granulation, Wurster fluidisation and fluidisation rotogranulation process can be used.

Further object of the present invention is a process for the preparation of controlled-release pharmaceutical composition containing carvedilol in layered pellets which comprises garnulating

a.) 5-50 weight%, preferably 10-40 weight%, more preferably 20-40 weight% of solid organic acid based on the total

weight of the pellet and optionally 5-20 weight%, preferably 10-40 weight%, more preferably 20-40 weight% of one ore more auxiliary agents are granulated in the usual way and the granules are dried, followed by

b.) coating the obtained pellet cores (1) according to the generally known method with an enteric layer (2) containing 0,5-5 weight%, preferably 1-5 weight%, more preferably 1-3 weight% of enteric polymer based on the total weight of the pellet Optionally one or more auxiliary agents may be used and if necessary, the obtained coated pellets are dried,

c.) coating of the pellets with a layer (3) containing a mixture of 5-30 weight%, preferably 10-20 weight%, more preferably 10-weight% of carvedilol based on the total weight of the pellet and 5-30 weight%, preferably 10-20 weight%, more preferably 10-15 weight% of water-soluble binder based on the total weight of the pellet. Optionally one or more auxiliary agents are usedf and if necessary the obtained coated pellets are dried, coating

d.) the layer (3) containing the carvedilol with a layer (4) containing a mixture 0.5-10 weight%, preferably 1-5 weight%, more preferably 1-3 weight% of water-soluble polymer based on the total weight of the pellet and 0.5-10 weight%, preferably 1-5 weight%, more preferably 1-3 weight% of the enteric polymer based on the total weight of the pellet.

The preparation of the multi-layered pellets can be accomplished as follows:

a.) The mixture of the solid organic acid and the other auxiliary agents are homogenized, the obtained mixture is granulated with water optionally containing further auxiliary agents, and if necessary, the granules are dried,

b.) the obtained pellet cores (1) are coated by spraying a suspension containing an enteric polymer dispersion and optionally other auxiliary agents on the core, than

c.) a further coating layer is applied by spraying an aqueous mixture of carvedilol, a water-soluble binding agent and optionally other auxiliary agents, or by subsequent application of the powdered carvedilol and the dispersion of the water-soluble binding agent, and optionally the obtained coated pellets are dried, then

d.) onto the layer containing carvedilol (3) a mixture of water-soluble film coating polymer, enteric film coating polymer and optionally other auxiliary agents dissolved in an organic solvent or an aqueous dispersion, are applied. The use of an alcoholic solution or an aqueous dispersion is preferred.

The preparation of the core can be accomplished with build-up granulation, centrifugal granulation, extrusion-spheronisation process, high-shear granulation or fluidisation rotogranulation process in which saturated or unsaturated di-and tricarboxylic acids containing 4-5 carbon atoms except fumaric acid. Preferably citric acid, tartaric acid, malic acid, maleic acid, most preferably succinic acid is used. Auxiliary agents generally used for the preparation of pellets in the pharmaceutical industry, especially lactose, starch, powdered cellulose, microcrystalline cellulose, colloid silicone dioxide, dimethylpolysiloxane, or mixtures thereof can be used as well in the preparation of the pellet core (1).

The surface of the core (1) receives enteric coating consists of methacrylic acid - methyl methacrylate copolymer, Methacrylic acid-ethyl acrylate copolymer, cellulose acetate phtalate, hydroxypropylmethylcellulose phtalate, hydroxypropylmethylcellulose acetate succinate, or polyvinylacetate-phtalate. Most advantageously methacrylic acid-ethyl acrylate copolymer is used. Besides the enteric polymers this layer can contain softeners and/or antiadhesive agents as auxiliary agents. Propylene glycol, triethylcitrate, polyethylene glycol or their mixtures can be used as softeners, talc, dimethylpolysiloxane or mixtures thereof can be used as antiadhesive agents. The layer can be prepared with build-up granulation process, centrifugal granulation process, Wurster-fluidisation, or fluidisation rotogranulation processes.

Hydroxypropylcellulose, hydroxypropylmethylcellulose, hydroxyethylcellulose, polyvinylpyrrolidone, vinylpyrrolidon/vinylacetate copolymer, polyvinylalcohol, polyethylene glycol, polyvinylalcohol-polyethylene glycol graft copolymer or their mixtures, preferably the mixture of hydroxypropylmethylcellulose and polyethylene glycol, are used as water-soluble binding agent, for the preparation of the layer containing carvedilol (3). The layer can be prepared by subsequent application of the aqueous solution of water-soluble binding agent and powdered carvedilol, or the spraying of a suspension containing powdered carvedilol and the water-soluble binding agent. The person skilled in the art can choose and optimise the most appropriate process among build-up granulation, centrifugal granulation, Wurster-fluidisation, fluidisation rotogranulation or other coating processes according to the state of the art.

If it is necessary further auxiliary agents can be used in the process for the preparation of the layer containing carvedilol (3).

The layer (4) on the surface of the layer containing carvedilol (3) which controls the dissolution of the active ingredient contains hydroxypropylcellulose, hydroxypropylmethylcellulose, hydroxyethylcellulose, polyvinylpyrrolidone, vinylpyrrolidon/vinylacetate copolymer, polyvinyl alcohol, polyethylene glycol, polyvinylalcohol-polyethylene glycol graft copolymer or their mixtures - preferably hydroxypropylcellulose for water-soluble polymer, and methacrylic acid - methyl methacrylate copolymer, methacrylic acid-ethyl acrylate copolymer, cellulose acetate phtalate, hydroxypropylmethylcellulose phtalate, hydroxypropyl methylcellulose acetate succinate or polyvinylacetate phtalate or their mixtures, preferably methacrylic acid-ethyl acrylate copolymer, for enteric polymer.

According to the most advantageous process of the present invention

a) 25-35 weight% of succinic acid based on the total weight of the pellets and 25-35 weight%, of microcrystalline cellulose are homogenized, and then granulated with a mixture of 0.4-1 weight% of dimethylpolysiloxane based on the total weight of the pellets and water, and the granules are dried,

b) the pellet cores obtained (1) are coated with 1-5 weight% of methacrylic acid-ethyl acrylate copolymer based on the total weight of the pellets, or optionally 0.1-0.35 weight% of propylene glycol and if necessary 0.1-0.15 weight% of antiadhesive agent is sprayed on the surface of the cores, then the pellets are optionally dried, then

c) the pellets are coated by spraying them a mixture of 10-15 weight% of carvedilol based on the total weight of the pellets and 10-15 weight% of a mixture of hydroxypropylmethylcellulose and polyethylene glycol. Optionally the mixture contains further auxiliary agents,

or,

the pellets are coated are coated with the subsequent application of 5-30 weight% of powdered carvedilol based on the total weight of the pellets, 5-30 weight% of a suspension of hydroxypropylmethylcellulose and polyethylene glycol. Optionally the mixture contains further auxiliary agents; and, if it is necessary the coated pellets obtained are dried.

d) Onto the layer containing carvedilol (3) an alcoholic mixture of 1-3 weight% of hydroxypropylcellulose and, 1-3 weight% of methacrylic acid-ethyl acrylate copolymer is sprayed.

The amount and the quality of the liquids used in the different steps of the preparation of layered pellets depend on the method and apparatuses chosen. The optimisation of the conditions is the duty of the person skilled in the art.

A further object of the present invention is to obtain a solid pharmaceutical dosage form and process, using pellets in which the pellets are mixed with auxiliary agents generally used in the pharmaceutical industry to form to galenic dosages.

The advantageous form is a tablet form which contains, based on the weight of the tablets, 40-90 weight %, preferably 60-90 weight %, more preferably 80-90 weight % of a pellet, 10-50 weight %, preferably 10-40 weight %, more preferably 10-20 weight % of excipients, 1-10 weight %, preferably 2-6 weight %, more preferably 3-5 weight % of the disintegrating agent, 2-10 weight %, preferably 3-8 weight %, more preferably 0.1-5 weight % of the binding agent, 0.1-2 weight %, preferably 0.1-1 weight %, of the lubricant. Tablets can be prepared throgh mixing the pellets with the filler and optionally with the disintegrating and binding agents and the lubricant. Finally they are pressed into their regular tablet form.

According to the present invention another advantageous solid dosage form is the capsule, which contains, based on the weight of the tablet 40-100 weight %, preferably 60-100 weight %, more preferably 80-100 weight % of the pellets, optionally 0.1-60 weight %, preferably 0.1-40 weight %, more preferably 0.1-20 weight % of filler, 0.1-10 weight %, preferably 0.1-10 weight %, more preferably 0.1-5 weight % of disintegrating agent, 0.1-10 weight %, preferably 0.1-5 weight %, more preferably 0.1-2 weight % of binding agent; and 0.1-2 weight %, or preferably 0.1-1 weight % of lubricant. Capsules can be formed by mixing the pellets with the filler and optionally with the disintegrating agent, the binding agent and the lubricant, and filling the composition into capsules.

Hard gelatine is also suitable for the preparation of capsules.

In vitro dissolution of the pellets in course of the tests for the invention was examined as follows:

The physiological parameters effective during the passing of the pharmaceutical composition through the gastrointestinal tract were taken into consideration. According to our actual knowledge the pH value of the contents of the stomach is about 1.2, and the pH value of the content of the intestinal tract is about 6.8. The small solid particles (smaller than 5 mm diameter) are emptied immediately (not later than 2 hours) when all particles are passed into the intestinal tract. Therefore the extreme time limits for dissolution of the active ingredient from the layered pellets according to the present invention having 1-2 mm diameter in acidic media under *in vivo* circumstances are zero or two hours. The dissolution can be seen independent from the physiological circumstances if the dissolution rate in a solution having pH 1.2 value for two hours followed by dissolution in a solution having a pH 6.8 value is similar to the dissolution rate of the pellets in a solution of pH 6.8 only.

The *in vitro* dissolution was measured as follows:

Apparatus: PhEUR I (basket), stirrer: 100 rpm, temperature: 37°C

Dissolution media:

A/ 0-24 hours pH 6.8 phosphate buffer

B/ 0-2 hours 0.1 M hydrochloric acid; 2-24 hours pH 6.8 phosphate buffer

| | |
|---|---|
| Active ingredient: | 6.25 mg/ 1 l |
| | 25mg/4 l |

Method for the analysis of the active ingredient: HPLC

The similarity factor ($F_2$) is calculated on the basis of the following formula

$$F_2 = 50\log\{100[1+(1/n)\sum_{t=1}^{n}(R_t-T_t)^{-2}]\}$$

wherein $R_t$ and $T_t$ are the measured dissolution rates of the compared pharmaceutical compositions (Reference and Test) at t moment.

The dissolution of the two compositions are seen similar if $F_2 > 50$.

The dissolution rates of the controlled release pellets containing carvedilol according to the present invention are shown in Figure 2.

| Dissolution | Sample | | | | | | | |
|---|---|---|---|---|---|---|---|---|
| Dissolution time (hours) | 1 hour | 2 hours | 4 hours | 6 hours | 8 hours | 12 hours | 16 hours | 24 hours |
| A/ (pH = 6.8) | 30.17 | 49.54 | 59.51 | 65.39 | 69.90 | 78.27 | 82.50 | 85.68 |
| B/ with pH exchange | 15.73 | 45.83 | 57.64 | 65.33 | 71.12 | 79.49 | 84.77 | 88.70 |

$F_2 = 63.81$

The controlled release pellets have several advantageous properties according to the present invention. The most important of them is that the pharmaceutical compositions containing the pellets obtained are multiparticulate controlled release compositions. The use of these multiparticulate compositions makes avoidable an extreme concentration of the active ingredient in the gastrointestinal tract.

Furthermore, these dosage forms ensure the relative uniformity of the absorption.

If the dissolution curves showing dissolution at pH 6.8 are compared with those which followed the pH rates of the stomach and then the intestinal tract, there is no significant difference between them. This means that the dissolution does not depend too much on the actual pH of the stomach.

It is a further advantage that the composition, that it has excellent dissolution properties, easily can be prepared from a carvedilol base and other auxiliary agents generally used in the pharmaceutical industry, and therefore it is not necessary to modify the known methods for the preparation of the active ingredient.

Additional advantage of the process is, that, according to the method invented, the pellets can be prepared by using the apparatuses and technological steps known in the pharmaceutical industry.

Pellets made according to the present invention facilitate the formulation of pharmaceutical compositions.

The preparation of the pellets according to the present invention are shown particularly as follows:

**Example 1**

Preparation of pellet cores containing solid organic acid

A mixture of 600 g of succinic acid and 600 g microcrystalline cellulose are homogenized for 5 minutes in a Glatt GPCG 3.1 fluidisation roto-granulator, then a mixture of 40 g 35% dimethylpolysiloxane emulsion and 1000 ml water is sprayed onto the homogenizate. The spraying rate of the solution is adjusted to 10 g/min. and the pressure of the spraying air is adjusted to 1.5 bar. The revolution of the rotor is 500 rpm during the wetting, then it is raised and kept at 900 rpm during the spinning-off. The volume velocity of the fluidisation air is kept at 80 $m^3$/hour for the first five minutes, then it is elevated and kept at 100 $m^3$/hour.

The dried pellets are separated to three different fractions by sieves having 0.63 mm and 1.25 mm thread distances. The yield of the sieve fraction between 0.63 mm and 1.25 mm is 96 weight %.

**Example 2**

Preparation of the acid release control layer

In a Glatt GPCG fluidisation equipment 500 g of pellets containing succinic acid and prepared according to Example 1 are coated with a dispersion of the mixture of 2 g of propyleneglycol, 15 g of methacrylic acid-ethacrylate copolymer and 210 g of water by using a Wurster attachment with a lower spraying rate. The spraying rate of the coating fluid is adjusted to 4-10 ml/minute, and the pressure of the spraying air to 1.5 bar. During the coating process the

fluidisation air stream is kept at 30-35 $m^3$/hour. The temperature of the inlet air is 36° C.

**Example 3**

Preparation of the layer containing carvedilol

In a Glatt GPCG fluidisation equipment, 500 g of pellets prepared according to Example 2 are coated with a dispersion of the mixture of 100 g carvedilol, and a suspension containing 90 g of hydroxypropyl-methylcellulose 10 g of polyethylene glycol 40 g of 35% dimethylpolysiloxane and 1000g of purified water using a Wurster attachment with lower spraying rate. The spraying rate of the coating fluid is adjusted to 6g/minute and the pressure of the spraying air is adjusted to 1.7 bar. During the coating process the fluidisation air stream is kept on 38 $m^3$/hour. The temperature of the inlet air stream is kept at 60° C.

**Example 4**

Preparation of the carvedilol dissolution control layer

In a Glatt GPCG fluidisation equipment 500 g of pellets prepared according to Example 3 are coated with a solution of 11 g of hydroxypropylcellulose, 13 g of methacrylic acid - ethylacrylate copolymer, 1.3 g of triethylcitrate in 350 g of high purity ethanol. The spraying rate of the coating fluid is adjusted to 6g/minute and the pressure of the spraying air to 1.7 bar. During the coating process the fluidisation air stream is kept at 35 $m^3$/hour. The temperature of the inlet air stream is kept at 60° C during the coating process.

## Claims

1. Pharmaceutical composition comprising carvedilol accordig to the formula

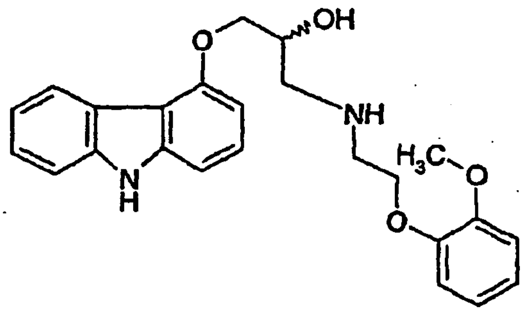

(I)

in a form of a layered pellet, wherein said pellet contains a core (1) containing a solid organic acid, an enteric layer (2) on the surface of the core, a layer (3) containing carvedilol and a water-soluble binding agent on the surface of the enteric layer (2), and an external dissolution controlling layer (4) containing a mixture of water-soluble polymer and an enteric polymer on the surface of the layer containing the active ingredient

2. Pharmaceutical composition containing carvedilol in a form of a layered pellet according to Claim 1, whrein the pellet has a core (1) containing 5-50 weight% of solid organic acid based on the total weight of the pellet, and optionally 5-50 weight% of one or more auxiliary agents, an enteric layer (2) on the surface of the core containing 0,5-10 weight% of enteric polymer based on the total weight of the pellet, and optionally further auxiliary agents, a further layer (3) on the surface of the enteric layer containing 5-30 weight% carvedilol based on the total weight of the pellet, and 5-30 weight% of water-soluble binder based on the total weight of the pellet and optionally further auxiliary agents, a further dissolution controlling layer (4) on the surface of the layer containing the active ingredient, containing a mixture of 0.5-10 weight% of water-soluble polymer based on the total weight of the pellet and 0.5-10 weight% of enteric polymer based on the total weight of the pellet and optionally further auxiliary agents.

3. Pharmaceutical composition containing carvedilol in a form of layered pellet according to the Claim 1, wherein the

pellet has a core (1) containing 10-40 weigh% of solid organic acid based on the total weight of the pellet and optionally 10-40 weight% of one or more auxiliary agents, an enteric layer (2) on the surface of the core containing 1-5 weight% of enteric polymer based on the total weight of the pellet and optionally further auxiliary agents, on the surface of the enteric layer a further layer (3) containing 10-20 weight% carvedilol based on the total weight of the pellet and 10-20 weight%, of water-soluble binder based on the weight of the total pellet and optionally further auxiliary agents, on the surface of the layer with the active ingredient a further dissolution controlling layer (4) containing a mixture of 1-5 weight% of water-soluble polymer based on the total weight of the pellet and 1-5 weight% of enteric polymer based on the total weight of the pellet and optionally further auxiliary agents.

**4.** Pharmaceutical composition containing carvedilol in a form of layered pellet according to the Claim 1, wherein the pellet has a core (1) containing 20-40 weight% of solid organic acid based on the total weight of the pellet and optionally 20-40 weight% of one or more auxiliary agents, an enteric layer (2) on the surface of the core containing 1-3 weight% of enteric polymer based on the total weight of the pellet and optionally further auxiliary agents, on the surface of the enteric layer a further layer (3) containing 10-15 weight% carvedilol based on the weight of the pellet and 10-15 weight%, of water-soluble binder based on the total weight of the pellet and optionally further auxiliary agents, on the surface of the layer with the active ingredient, a further dissolution controlling layer (4) containing a mixture of 1-3 weight% of water-soluble polymer based on the total weight of the pellet and 1-3 weight% of enteric polymer based on the total weight of the pellet and optionally further auxiliary agents.

**5.** Pharmaceutical composition comprising carvedilol in a form of layered pellet according to any of the Claims 1-4 **characterised in that** the core contains a physiologically inert solid organic acid, preferably saturated or unsaturated, di- or tricarboxylic acid having 4-5 carbon atoms except fumaric acid, more preferably citric acid, tartaric acid, malic acid, maleic acid, succinic acid and most preferably succinic acid.

**6.** Pharmaceutical composition containing carvedilol in a form of layered pellet according to any of the Claims 1-4 whrerein the auxiliary agents used for preparation of pellet core (1) are generally accepted for the preparation of pellets in the pharmaceutical industry, preferably lactose, starch, powdered cellulose, microcrystalline cellulose, colloid silicon dioxid, dimethylpolysiloxane or mixture thereof, most preferably microcrystalline cellulose and optionally dimethylpolysiloxane.

**7.** Pharmaceutical composition containing carvedilol in a form of layered pellet according to any of the Claims 1-6 , wherein the enteric layer (2) on the surface of the pellet core (1) contains enteric coating polymer, preferably methacrylic acid-ethyl acrylate copolymer, methacrylic acid - methyl methacrylate copolymer, cellulose acetate phtalate, hydroxypropylmethylcellulose acetate succinate or polyvinylacetate-phtalate, more preferably methacrylic acid-ethyl acrylate copolymer.

**8.** Pharmaceutical composition containing carvedilol in a form of layered pellet according to any of the Claims 1-6, wherein the enteric layer (2) on the surface of the pellet core (1) contains further auxiliary agents such as softeners or optionally antiadhesive agents.

**9.** Pharmaceutical composition containing carvedilol in the form of layered pellet according to the Claim 8, wherein the enteric layer (2) contains propylene glycol, triethylcitrate, polyethylene glycol or mixtures thereof as softener.

**10.** Pharmaceutical composition containing carvedilol in the form of layered pellet according to any of the Claims 8 or 9, wherein the enteric layer (2) contains talc, dimethylpolysiloxane or mixtures thereof as antiadhesive agents.

**11.** Pharmaceutical composition containing carvedilol in the form of layered pellet according to any of the Claims 1-10, wherein the layer comprising carvedilol (3) contains hydroxypropylcellulose, hydroxypropylmethylcellulose, hydroxyethylcellulose, polyvinylpyrrolidone, vinylpyrrolidone/vinylacetate copolymer, polyvinylalcohol, polyethylene glycol polyvinylalcohol-polyethylene glycol graft copolymer or mixtures thereof preferably a mixture of hydroxypropylmethylcellulose and polyethylene glycol as water-soluble binding agents.

**12.** Pharmaceutical composition containing carvedilol in the form of layered pellet according to any of the Claims 1-11, wherein the layer (4) on the surface of the layer comprising carvedilol (3) contains hydroxypropylcellulose, hydroxypropylmethylcellulose, hydroxyethylcellulose, polyvinylpyrrolidone, vinylpyrrolidon/vinylacetate copolymer, polyvinylalcohol, polyethylene glycol, polyvinylalcohol-polyethylene glycol graft copolymer or mixtures thereof preferably hydroxypropylcellulose as water-soluble film coating agent

**13.** Pharmaceutical composition containing carvedilol in the form of layered pellet according to any of Claims 1-12, wherein the layer (4) on the surface of the layer comprising carvedilol (3) contains methacrylic acid-ethyl acrylate copolymer, methacrylic acid - methyl methacrylate copolymer, cellulose acetate phtalate, hydroxymethylcellulose phtalate, hydroxypropylmethylcellulose acetate succinate or polyvinylacetate phtalate, or mixtures thereof preferably methacrylic acid-ethyl acrylate copolymer as enteric film coating agent.

**14.** Pharmaceutical composition containing carvedilol in the form of layered pellet according to the Claim 1, wherein the pellet has a core (1) containing 25-35 weight% of succinic acid based on the total weight of the pellet and 25-35 weight% of microcrystalline cellulose and 0.4-1 weight % of dimethylpolysiloxane, an enteric layer (2) on the surface of the core containing 1-5 weight% of methacrylic acid-ethyl acrylate copolymer and optionally further 0.1-0.35 weight % of propylene glycol, on the surface of the enteric layer a further layer (3) containing 10-15 weight% carvedilol based on the total weight of the pellet and 10-15 weight%, of a mixture of hydroxypropylmethylcellulose and polyethylene glycol based on the total weight of the pellet, on the surface of the layer containing the active ingredient, and a further layer (4) containing a mixture of 1-3 weight% of hydroxypropylcellulose based on the total weight of the pellet and 1-3 weight% of methacrylic acid-ethyl acrylate copolymer based on the total weight of the pellet.

**15.** Controlled release pharmaceutical solid dosage form which contains pellets according to any of the Claims 1-14 and optionally other auxiliary agents used in the pharmaceutical industry.

**16.** Controlled release pharmaceutical solid dosage form according to Claim 15 **characterized in that** the dosage form is tablet or capsule.

**17.** Solid dosage form according to Claim 16, **characterized in that** the tablets contain 40-90 weight %, preferably 60-90 weight %, more preferably 80-90 weight % of pellets, 10-50 weight %, preferably 10-40 weight %, more preferably 10-20 weight % of filler, 1-10 weight %, preferably 2-6 weight %, more preferable 3-5 weight % of disintegrating agent, 2-10 weight %, preferably 3-8 weight %, more preferably 0.1-5 weight % of binding agent, 0.1-1 weight %, preferably 0.1-1 weight %, of lubricant.

**18.** Solid dosage form according to Claim 16 **characterized in that** the capsules contain 40-100 weight %, preferably 60-100 weight %, more preferably 80-100 weight % of pellets, optionally 0.1-60 weight %, preferably 0.1-40 weight %, more preferably 0.1-20 weight % of filler, 0.1-10 weight %, preferably 0.1-5 weight % of disintegrating agent, 0.1-2 weight %, preferably 0.1-1 weight %, of lubricant

**19.** Capsules according to Claim 18 **characterized in that** hard gelatine capsules are used.

**20.** Controlled release pharmaceutical solid dosage form containing pellets according to any of the Claims 15-19, **characterized in that** pharmaceutically acceptable filler, preferably lactose, starch, powdered cellulose, microcrystalline cellulose, colloid silicon dioxid, dimethylpolysiloxane or mixtures thereof, more preferably microcrystalline cellulose, colloid silicon dioxid, dimethylpolysiloxane or mixtures thereof are used as fillers, pharmaceutically acceptable binding agent, preferably carboxymethylcellulose or low-substituted hydroxypropylcellulose, pharmaceutically accepted disintegrating agent, preferably sodium-carboximethylcellulose, crosslinked polyvinylpyrrolidone, sodium carboxymethylstarch, or low-substituted hydroxypropylcellulose, more preferably crospovidone, pharmaceutically acceptable lubricants, preferably calcium and magnesium stearate, glyceryl behanate, stearic acid talc or hydrogenated vegetable oils.

**21.** Process for the preparation of a pharmaceutical composition containing carvedilol in a form of layered pellets, **characterised in that**

a.) a solid organic acid and optionally one or more excipients are granulated in a known manner, the obtained pellet core (1)
b.) is coated with an enteric coating polymer in a known manner, the obtained coated pellets are optionally dried,
c.) the surface of the enteric layer (2) is coated with a mixture of carvedilol and a water-soluble binding agent optionally using other auxiliary agents in a manner known, optionally dried,
d.) the obtained coated pellets are coated in a known manner with a mixture of a water-soluble and an enteric coating polymer.

**22.** Process for the preparation of a pharmaceutical composition containing carvedilol in a form of layered pellets according to Claim 21 **characterised in that**

a.) 5-50 weight % of solid organic acid based on the total weight of the pellets and optionally 5-50 weight % of one or more auxiliary agents based on the total weight of the pellets are granulated in a known manner, the obtained pellet cores (1) are dried, and
b.) coated with 0.5-10 weight % enteric film coating polymer based on the total weight of the pellets and optionally one or more auxiliary agents preferably softeners and antiadhesive agents in a usual manner and optionally dried,
c.) the surface of the enteric layer (2) is coated with a mixture of 5-30 weight % of carvedilol based on the total weight of the pellets and 5-30 weight % of water-soluble binding agent and optionally using other auxiliary agents in a usual manner, the obtained coated pellets are optionally dried, then
d.) the layer containing carvedilol (3) is coated with a dissolution controlling layer (4) in a usual manner with a mixture of 0.5-10 weight % of enteric coating polymer and 0.5-10 weight % of water-soluble polymer based on the total weight of the pellets.

**23.** Process for the preparation of a pharmaceutical composition containing carvedilol in a form of layered pellets according to Claim 21 **characterised in that**

a.) the total weight of the pellets and optionally 10-40 weight % of one or more excipients based on the total weight of the pellets are granulated in a known manner, the obtained pellet cores (1) are dried, and
b.) coated with 1-5 weight % enteric film coating polymer based on the total weight of the pellets and optionally one or more auxiliary agents preferably softeners and antiadhesive agents in a usual manner and optionally dried,
c.) the surface of the enteric layer (2) is coated with a mixture of 10-20 weight % of carvedilol based on the total weight of the pellets and 10-20 weight % of water-soluble binding agent and optionally using other auxiliary agents in a usual manner, the obtained coated pellets are optionally dried, then
d.) the layer containing carvedilol (3) is coated with a dissolution controlling layer (4) in a usual manner with a mixture of 1-5 weight % of enteric coating polymer and 1-5 weight % of water-soluble polymer based on the total weight of the pellets.

**24.** Process for the preparation of a pharmaceutical composition containing carvedilol in a form of layered pellets according to Claim 21 **characterised in that**

a.) 20-40 weight % of solid organic acid based on the total weight of the pellets and optionally 20-40 weight % of one or more auxiliary agents based on the total weight of the pellets are granulated in a known manner, the obtained pellet cores (1) are dried, and
b.) coated with 1-3 weight % enteric film coating polymer based on the total weight of the pellets and optionally one or more auxiliary agents preferably softeners and antiadhesive agents in a known manner and optionally dried,
c.) the surface of the enteric layer (2) is coated with a mixture of 10-15 weight % of carvedilol based on the total weight of the pellets and 10-15 weight % of water-soluble binding agent and optionally using other auxiliary agents in a known manner, the obtained coated pellets are optionally dried, then
d.) the layer containing carvedilol (3) is coated with a dissolution controlling layer (4) in a known manner with a mixture of 1-3 weight % of enteric coating polymer and 1-3 weight % of water-soluble polymer based on the total weight of the pellets.

**25.** Process according to any of the Claims 21-24 **characterized in that**

a.) the organic acid and the auxiliary agents are homogenized, then the obtained homogenized mixture is granulated with water which can optionally contain further auxiliary agents, and the obtained granules are dried,
b.) the obtained pellet cores (1) are coated by spraying a dispersion of a mixture of enteric film coating polymer dispersion and optionally other auxiliary agents on the cores, the obtained coated pellets are dried if necessary,
c.) the enteric coated pellets are sprayed with a suspension of a mixture of carvedilol and a water-soluble binding agent, optionally other auxiliary agents are applied to the enteric coated pellets or, by subsequent application of the powdered carvedilol and the dispersion of the water-soluble binding agent, and optionally the obtained coated pellets are dried, then
d.) a mixture of enteric coating polymer, water-soluble polymer and optionally other auxiliary agents are sprayed in a form of solution or dispersion containing water or organic solvents, preferably in form of alcoholic solution or dispersion onto the layer containing carvedilol.

**26.** Process according to any of the Claims 21-25 **characterized in that** the organic acid used for the preparation of the pellet cores (1) are physiologically inert solid organic acids except fumaric acid, preferably saturated and unsaturated di- and tricarboxylic acids having 4-5 carbon atoms, more preferably citric acid, tartaric acid, malic acid, maleic acid, succinic acid and most preferably succinic acid.

**27.** Process according to any of the Claims 21-26 wherein the used auxiliary agents for the preparation of the pellet cores (1) are used in the pharmaceutical industry, preferably lactose, starch, powdered cellulose, microcrystalline cellulose, colloid silicon dioxid, dimethylpolysiloxane or mixtures thereof, more preferably microcrystalline cellulose, colloid silicon dioxid, dimethylpolysiloxane or mixtures thereof.

**28.** Process according to any of the Claims 21-27 **characterized in that** the surface of the pellet core (1) is coated with enteric film coating polymer or polymers, preferably methacrylic acid-ethyl acrylate copolymer, methacrylic acid - methyl methacrylate copolymer, cellulose acetate phtalate, hydroxypropylmethylcellulose phtalate, hydroxypropylmethylcellulose acetate succinate or polyvinylacetate-phtalate, more preferably methacrylic acid-ethyl acrylate copolymer.

**29.** Process according to Claim 28 **characterized in that** other auxiliary agents, optionally softeners and antiadhesive compounds are also used in the process of the application the enteric coating layer (2) onto the pellet core (1).

**30.** Process according to Claim 29 **characterized in that** propylene glycol, triethylcitrate, polyethylene glycol or mixture thereof are used as softener in the process of the application the enteric coating layer (2) onto the pellet core (1).

**31.** Process according to Claim 29 **characterized in that** talc, dimethylpolysiloxane or mixture thereof are used as antiadhesive agent in the process of the application the enteric coating layer (2) onto the pellet core (1).

**32.** Process according to any of the claims 21-31 **characterized in that** the layer containing carvedilol (3) is prepared by using hydroxypropylcellulose, hydroxypropylmethylcellulose, hydroxyethylcellulose, polyvinylpyrrolidone vinylpyrrolidon/vinylacetate copolymer, polyvinylalcohol, polyethylene glycol, polyvinylalcohol-polyethylene glycol graft copolymer or mixtures thereof, preferably a mixture of hydroxypropylcellulose and polyethylene glycol as water-soluble film coating agent.

**33.** Process according to Claim 32 **characterized in that** further auxiliary agents are used.

**34.** Process according to any of the Claims of 21-33 **characterised in that** the layer containing carvedilol (3) is prepared by the subsequent application of powdered carvedilol and the water-soluble binding agent or, with spraying of a suspension containing a mixture of the carvedilol and the aqueous mixture of the water-soluble binding agent.

**35.** Process according to any of the Claims 21-34 **characterized in that** hydroxypropylcellulose, hydroxypropylmethylcellulose, hydroxyethylcellulose, polyvinylpyrrolidone, vinylpyrrolidon/vinylacetate copolymer, polyvinylalcohol, polyethylene glycol, polyvinylalcohol-polyethylene glycol graft copolymer or mixtures thereof preferably hydroxypropylcellulose are used as water-soluble film coating compounds for the preparation of the external layer (4) on the surface of the layer containing carvedilol (3).

**36.** Process according to any of the Claims 21-34 **characterized in that** methacrylic acid-ethyl acrylate copolymer, methacrylic acid - methyl methacrylate copolymer, cellulose acetate phtalate, hydroxymethylcellulose-phtalate, hydroxypropyl-methylcellulose-acetate-succinate or polyvinylacetate-phtalate, or mixtures thereof, preferably methacrylic acid - ethylacrylate copolymer are used as water-soluble film coating compound for the preparation of the external layer (4) on the surface of the layer containing carvedilol (3).

**37.** Process for the preparation of a pharmaceutical composition containing carvedilol in a form of multi-layered pellets according to Claim 21 **characterised in that**

a.) 25-35 weight % of succinic acid and optionally 25-35 weight % of microcrystalline cellulose are granulated with water containing 0.4-1 weight % of dimethylpolysiloxane based on the total weight of the pellets, the obtained pellet cores (1) are dried, and
b.) coated with 1-5 weight % of methacrylic acid-ethyl acrylate copolymer, optionally with a mixture of 0.1-0.35 weight % of propylene glycol and 0.1-0.15 weight % of antiadhesive agent based on the total weight of the pellets by spraying onto the surface of the cores (1), the obtained coated pellets are optionally dried, then
c.) onto the surface of the enteric layer (2) a further layer is applied by spraying the coated pellets with a mixture of 10-15 weight % of carvedilol based on the weight and 10-15 weight % of a mixture of hydroxypropylmethylcellulose, polyethylene glycol and optionally further auxiliary agents
or,
the layer is prepared by the subsequent application of 5-30 weight % of powdered carvedilol and 5-30 weight % of the mixture of hydroxymethylcellulose polyethylene glycol and optionally further auxiliary agents,
the obtained pellets are optionally dried, then
d.) the obtained layer containing carvedilol (3) is coated by spraying with an alcoholic mixture of 1-3 weight % of methacrylic acid-ethyl acrylate copolymer and 1-3 weight % of hydroxypropylcellulose based on the total weight of the pellet.

**38.** Process for the preparation of controlled release solid dosage form according to any of the Claims 1-14 **characterized in that** the pellets are optionally mixed with auxiliary agents generally used in the pharmaceutical industry and then transformed to the galenic form.

**39.** Process for the preparation of controlled release solid dosage form according to Claim 38, wherein 40-90 weight %, preferably 60-90 weight %, more preferably 80-90 weight % of pellets according to any of the Claims 1-14, 10-50 weight %, preferably 10-40 weight %, more preferably 10-20 weight % of the filler, optionally 1-10 weight %, preferably 2-6 weight % of the disintegrating agent, 2-10 weight %, preferably 3-8 weight %, more preferably 0.1-5 weight % of the binding agent and optionally 0.1-1 weight % lubricant are mixed and pressed into tablets.

**40.** Process for the preparation of controlled release solid dosage form according to Claim 38 wherein 40-100 weight %, preferably 60-100 weight %, more preferably 80-100 weight % of the pellets according to any of the Claims 1-14, optionally 0.1-60 weight %, preferably 0.1-20 weight %, more preferably 0.1-10 weight % of the filler, optionally 0.1-20 weight %, preferably 0.1-10 weight %, more preferably 0.1-5 weight % of the disintegrating agent, 2-10 weight %, preferably 3-8 weight %, more preferably 0.1-5 weight % of the binding agent and optionally 0.1-2 weight %, preferably 0.1-1 weight % of the lubricant are mixed and filled into capsules.

**41.** Process for the preparation of capsules according to Claim 40 **characterized in that** hard gelatine capsules are used.

**42.** Process for the preparation of dosage forms according to any of the Claims of 38-41 **characterised in that** the generally acceptable excipients, preferably lactose, starch, powdered cellulose, microcrystalline cellulose, colloid silicon dioxid, dimethylpolysiloxane or mixtures thereof, most preferably microcrystalline cellulose, colloid silicon dioxid, dimethylpolysiloxane or mixtures thereof are used, any pharmaceutically acceptable binding agent, preferably polyvinylpyrrolidone, hydroxypropylcellulose are used, any of the pharmaceutically accepted disintegrating agents, preferably sodium-carboxymethylcellulose, crosslinked polyvinylpyrrolidone, sodium carboxymethylstarch, or low-substituted hydroxypropylcellulose, more preferably crospovidone are used, any of the pharmaceutically acceptable lubricants, preferably calcium and magnesium stearate, glyceryl behanate, stearic acid talc or hydrogenated vegetable oils can be used.

## Patentansprüche

**1.** Pharmazeutische Zusammensetzung, umfassend Carvedilol gemäß der Formel:

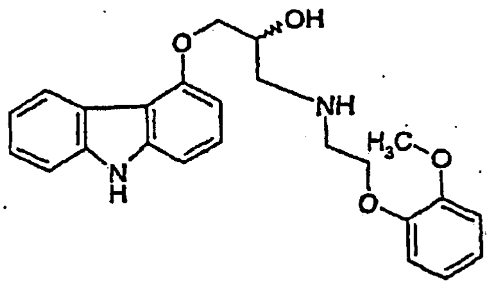

(I)

in einer Form eines geschichteten Pellets, wobei das Pellet einen Kern (1), enthaltend eine feste organische Säure, eine enterische Schicht (2) auf der Oberfläche des Kerns, eine Schicht (3), enthaltend Carvedilol und ein wasserlösliches Bindemittel auf der Oberfläche der enterischen Schicht (2), und eine äußere, die Auflösung regulierende Schicht (4), enthaltend eine Mischung von wasserlöslichem Polymer und einem enterischen Polymer auf der Oberfläche der den aktiven Bestandteil enthaltenden Schicht, enthält.

2. Pharmazeutische Zusammensetzung, enthaltend Carvedilol in einer Form eines geschichteten Pellets gemäß Anspruch 1, wobei das Pellet einen Kern (1), enthaltend 5 - 50 Gew.-% einer festen organischen Säure auf Basis des Gesamtgewichts des Pellets und gegebenenfalls 5 - 50 Gew.-% eines oder mehrerer Hilfsmittel, eine enterische Schicht (2) auf der Oberfläche des Kerns, enthaltend 0,5 - 10 Gew.-% enterisches Polymer auf Basis des Gesamtgewichts des Pellets, und gegebenenfalls weitere Hilfsmittel, eine weitere Schicht (3) auf der Oberfläche der enterischen Schicht, enthaltend 5 - 30 Gew.-% Carvedilol auf Basis des Gesamtgewichts des Pellets und 5 - 30 Gew.-% wasserlösliches Bindemittel auf Basis des Gesamtgewichts des Pellets und gegebenenfalls weitere Hilfsmittel, eine weitere die Auflösung regulierende Schicht (4) auf der Oberfläche der den aktiven Bestandteil enthaltenden Schicht, enthaltend eine Mischung von 0,5 - 10 Gew.-% wasserlöslichem Polymer auf Basis des Gesamtgewichts des Pellets und 0,5 - 10 Gew.-% enterischem Polymer auf Basis des Gesamtgewichts des Pellets, und gegebenenfalls weitere Hilfsmittel aufweist.

3. Pharmazeutische Zusammensetzung, enthaltend Carvedilol in einer Form eines geschichteten Pellets gemäß Anspruch 1, wobei das Pellet einen Kern (1), enthaltend 10 - 40 Gew.-% einer festen organischen Säure auf Basis des Gesamtgewichts des Pellets und gegebenenfalls 10 - 40 Gew.-% eines oder mehrerer Hilfsmittel(s), eine enterische Schicht (2) auf der Oberfläche des Kerns, enthaltend 1 - 5 Gew.-% enterisches Polymer auf Basis des Gesamtgewichts des Pellets, und gegebenenfalls weitere Hilfsmittel auf der Oberfläche der enterischen Schicht eine weitere Schicht (3), enthaltend 10 - 20 Gew.-% Carvedilol auf Basis des Gesamtgewichts des Pellets und 10 - 20 Gew.-% wasserlösliches Bindemittel auf Basis des Gewichts des Gesamtpellets und gegebenenfalls weitere Hilfsmittel, auf der Oberfläche der Schicht mit dem aktiven Bestandteil eine weitere die Auflösung regulierende Schicht (4), enthaltend eine Mischung von 1 - 5 Gew.-% wasserlöslichem Polymer auf Basis des Gesamtgewichts des Pellets und 1 - 5 Gew.-% enterischem Polymer auf Basis des Gesamtgewichts des Pellets und gegebenenfalls weitere Hilfsmittel aufweist.

4. Pharmazeutische Zusammensetzung, enthaltend Carvedilol in einer Form eines geschichteten Pellets gemäß Anspruch 1, wobei das Pellet einen Kern (1), enthaltend 20 - 40 Gew.-% einer festen organischen Säure auf Basis des Gesamtgewichts des Pellets und gegebenenfalls 20 - 40 Gew.-% eines oder mehrerer Zusatzmittel, eine enterische Schicht (2) auf der Oberfläche des Kerns, enthaltend 1 - 3 Gew.-% enterisches Polymer auf Basis des Gesamtgewichts des Pellets, und gegebenenfalls weitere Hilfsmittel, auf der Oberfläche der enterischen Schicht eine weitere Schicht (3), enthaltend 10 - 15 Gew.-% Carvedilol auf Basis des Gewichts des Pellets und 10 - 15 Gew.-% wasserlösliches Bindemittel auf Basis des Gesamtgewichts des Pellets und gegebenenfalls weitere Hilfsmittel, auf der Oberfläche der Schicht mit dem aktiven Bestandteil eine weitere die Auflösung regulierende Schicht (4), enthaltend eine Mischung von 1 - 3 Gew.-% wasserlöslichem Polymer auf Basis des Gesamtgewichts des Pellets und 1 - 3 Gew.-% enterischem Polymer auf Basis des Gesamtgewichts des Pellets und gegebenenfalls weitere Hilfsmittel aufweist.

5. Pharmazeutische Zusammensetzung, umfassend Carvedilol in einer Form eines geschichteten Pellets gemäß einem beliebigen der Ansprüche 1 - 4, **dadurch gekennzeichnet, dass** der Kern eine physiologisch inerte, feste organische Säure, vorzugsweise gesättigte oder ungesättigte Di- oder Tricarbonsäure mit 4 - 5 Kohlenstoffatomen, ausgenommen Fumarsäure, stärker bevorzugt Citronensäure, Weinsäure, Äpfelsäure, Maleinsäure, Bernsteinsäure und am meisten bevorzugt Bernsteinsäure enthält.

6. Pharmazeutische Zusammensetzung, enthaltend Carvedilol in einer Form eines geschichteten Pellets gemäß einem beliebigen der Ansprüche 1 - 4, wobei die für die Herstellung des Pelletkerns (1) verwendeten Hilfsmittel allgemein für die Herstellung von Pellets in der pharmazeutischen Industrie akzeptiert sind, vorzugsweise Lactose, Stärke, pulverförmige Cellulose, mikrokristalline Cellulose, kolloidales Siliciumdioxid, Dimethylpolysiloxan oder eine Mischung davon, am meisten bevorzugt mikrokristalline Cellulose und gegebenenfalls Dimethylpolysiloxan.

7. Pharmazeutische Zusammensetzung, enthaltend Carvedilol in einer Form eines geschichteten Pellets gemäß einem beliebigen der Ansprüche 1 - 6, wobei die enterische Schicht (2) auf der Oberfläche des Pelletkerns (1) enterisches Beschichtungspolymer, vorzugsweise Methacrylsäure-Ethylacrylat-Copolymer, Methacrylsäure-Methylmethacrylat-Copolymer, Celluloseacetatphthalat, Hydroxypropylmethylcelluloseacetatsuccinat oder Polyvinylacetatphthalat, stärker bevorzugt Methacrylsäure-Ethylacrylat-Copolymer enthält.

8. Pharmazeutische Zusammensetzung, enthaltend Carvedilol in einer Form eines geschichteten Pellets gemäß einem beliebigen der Ansprüche 1 - 6, wobei die enterische Schicht (2) auf der Oberfläche des Pelletkerns (1) weitere Hilfsmittel, wie Weichmacher oder gegebenenfalls Antihaftmittel, enthält.

9. Pharmazeutische Zusammensetzung, enthaltend Carvedilol in der Form eines geschichteten Pellets gemäß Anspruch 8, wobei die enterische Schicht (2) Propylenglykol, Triethylcitrat, Polyethylenglykol oder Mischungen davon als Weichmacher enthält.

10. Pharmazeutische Zusammensetzung, enthaltend Carvedilol in der Form eines geschichteten Pellets gemäß einem beliebigen der Ansprüche 8 oder 9, wobei die enterische Schicht (2) Talk, Dimethylpolysiloxan oder Mischungen davon als Antihaftmittel enthält.

11. Pharmazeutische Zusammensetzung, enthaltend Carvedilol in der Form eines geschichteten Pellets gemäß einem beliebigen der Ansprüche 1 - 10, wobei die Carvedilol umfassende Schicht (3) Hydroxypropylcellulose, Hydroxypropylmethylcellulose, Hydroxyethylcellulose, Polyvinylpyrrolidon, Vinylpyrrolidon/Vinylacetat-Copolymer, Polyvinylalkohol, Polyethylenglykol, Polyvinylalkohol-Polyethylenglykol-Pfropfcopolymer oder Mischungen davon, vorzugsweise eine Mischung von Hydroxypropylmethylcellulose und Polyethylenglykol als wasserlösliche Bindemittel enthält.

12. Pharmazeutische Zusammensetzung, enthaltend Carvedilol in der Form eines geschichteten Pellets gemäß einem beliebigen der Ansprüche 1 - 11, wobei die Schicht (4) auf der Oberfläche der Carvedilol umfassenden Schicht (3) Hydroxypropylcellulose, Hydroxypropylmethylcellulose, Hydroxyethylcellulose, Polyvinylpyrrolidon, Vinylpyrrolidon/ Vinylacetat-Copolymer, Polyvinylalkohol, Polyethylenglykol, Polyvinylalkohol-Polyethylenglykol-Pfropfcopolymer oder Mischungen davon, vorzugsweise Hydroxypropylcellulose, als Beschichtungsmittel in Form eines wasserlöslichen Films enthält.

13. Pharmazeutische Zusammensetzung, enthaltend Carvedilol in der Form eines geschichteten Pellets gemäß einem beliebigen der Ansprüche 1 - 12, wobei die Schicht (4) auf der Oberfläche der Carvedilol umfassenden Schicht (3) Methacrylsäure-Ethylacrylat-Copolymer, Methacrylsäure-Methylmethacrylat-Copolymer, Celluloseacetatphthalat, Hydroxymethylcellulosephthalat, Hydroxypropylmethylcelluloseacetatsuccinat oder Polyvinylacetatphthalat oder Mischungen davon, vorzugsweise Methacrylsäure-Ethylacrylat-Copolymer als Beschichtungsmittel in Form eines enterischen Films enthält.

14. Pharmazeutische Zusammensetzung, enthaltend Carvedilol in der Form eines geschichteten Pellets gemäß Anspruch 1, wobei das Pellet einen Kern (1), enthaltend 25 - 35 Gew.-% Bernsteinsäure auf Basis des Gesamtgewichts des Pellets und 25 - 35 Gew.-% mikrokristalline Cellulose und 0,4 - 1 Gew.-% Dimethylpolysiloxan, eine enterische Schicht (2) auf der Oberfläche des Kerns, enthaltend 1 - 5 Gew.-% Methacrylsäure-Ethylacrylat-Copolymer und gegebenenfalls weiter 0,1 - 0,35 Gew.-% Propylenglykol, auf der Oberfläche der enterischen Schicht eine weitere Schicht (3), enthaltend 10 - 15 Gew.-% Carvedilol auf Basis des Gesamtgewichts des Pellets und 10 - 15 Gew.-% einer Mischung von Hydroxypropylmethylcellulose und Polyethylenglykol auf Basis des Gesamtgewichts des Pellets auf der Oberfläche der den aktiven Bestandteil enthaltenden Schicht, und eine weitere Schicht (4), enthaltend eine Mischung von 1 - 3 Gew.-% Hydroxypropylcellulose auf Basis des Gesamtgewichts des Pellets und von 1 - 3 Gew.-% Methacrylsäure-Ethylacrylat-Copolymer auf Basis des Gesamtgewicht des Pellets, aufweist.

15. Pharmazeutische feste Dosierungsform zur kontrollierten Freisetzung, welche Pellets gemäß einem beliebigen der Ansprüche 1 - 14 und gegebenenfalls andere in der pharmazeutischen Industrie verwendete Hilfsmittel enthält.

16. Pharmazeutische feste Dosierungsform zur kontrollierten Freisetzung gemäß Anspruch 15, **dadurch gekennzeichnet, dass** die Dosierungsform eine Tablette oder Kapsel ist.

17. Feste Dosierungsform gemäß Anspruch 16, **dadurch gekennzeichnet, dass** die Tabletten 40 - 90 Gew.-%, vorzugsweise 60 - 90 Gew.-%, stärker bevorzugt 80 - 90 Gew.-% Pellets, 10 - 50 Gew.-%, vorzugsweise 10 - 40 Gew.-%, stärker bevorzugt 10 - 20 Gew.-% Füllstoff, 1 - 10 Gew.-%, vorzugsweise 2 - 6 Gew.-%, stärker bevorzugt 3 - 5 Gew.-% Sprengmittel, 2 - 10 Gew.-%, vorzugsweise 3 - 8 Gew.-%, stärker bevorzugt 0,1 - 5 Gew.-% Bindemittel, 0,1 - 1 Gew.-%, vorzugsweise 0,1 - 1 Gew.-% Gleitmittel enthalten.

18. Feste Dosierungsform gemäß Anspruch 16, **dadurch gekennzeichnet, dass** die Kapseln 40 - 100 Gew.-%, vorzugsweise 60 - 100 Gew.-%, stärker bevorzugt 80 - 100 Gew.-% Pellets, gegebenenfalls 0,1 - 60 Gew.-%, vorzugsweise 0,1 - 40 Gew.-%, stärker bevorzugt 0,1 - 20 Gew.-% Füllstoff, 0,1 - 10 Gew.-%, vorzugsweise 0,1 - 5 Gew.-%, Sprengmittel, 0,1 - 2 Gew.-%, vorzugsweise 0,1 - 1 Gew.-% Gleitmittel enthalten.

**19.** Kapseln gemäß Anspruch 18, **dadurch gekennzeichnet, dass** harte Gelatinekapseln verwendet werden.

**20.** Pharmazeutische feste Dosierungsform zur kontrollierten Freisetzung, welche Pellets gemäß einem beliebigen der Ansprüche 15 - 19 enthält, **dadurch gekennzeichnet, dass** pharmazeutisch annehmbarer Füllstoff, vorzugsweise Lactose, Stärke, pulverförmige Cellulose, mikrokristalline Cellulose, kolloidales Siliciumdioxid, Dimethylpolysiloxan oder Mischungen davon, stärker bevorzugt mikrokristalline Cellulose, kolloidales Siliciumdioxid, Dimethylpolysiloxan oder Mischungen davon als Füllstoffe verwendet werden, pharmazeutisch annehmbares Bindemittel, vorzugsweise Carboxymethylcellulose oder niedrig substituierte Hydroxypropylcellulose, pharmazeutisch akzeptiertes Sprengmittel, vorzugsweise Natriumcarboxymethylcellulose, vernetztes Polyvinylpyrrolidon, Natriumcarboxymethylstärke oder niedrig substituierte Hydroxypropylcellulose, stärker bevorzugt Crospovidon, pharmazeutisch annehmbare Gleitmittel, vorzugsweise Calcium- und Magnesiumstearat, Glycerylbehanat, Stearinsäuretalk oder hydrierte pflanzliche Öle.

**21.** Verfahren für die Herstellung einer Carvedilol enthaltenden pharmazeutischen Zusammensetzung in einer Form von geschichteten Pellets, **dadurch gekennzeichnet, dass**

a) eine feste organische Säure und gegebenenfalls ein oder mehrere Arzneimittelträger in einer bekannten Weise granuliert werden, der erhaltene Pelletkern (1)
b) mit einem enterischen Beschichtungspolymer in einer bekannten Weise beschichtet wird, die erhaltenen beschichteten Pellets gegebenenfalls getrocknet werden,
c) die Oberfläche der enterischen Schicht (2) mit einer Mischung von Carvedilol und einem wasserlöslichen Bindemittel gegebenenfalls unter Verwendung anderer Hilfsmittel in einer bekannten Weise beschichtet wird, gegebenenfalls getrocknet wird,
d) die erhaltenen beschichteten Pellets in einer bekannten Weise mit einer Mischung eines wasserlöslichen und eines enterischen Beschichtungspolymers beschichtet werden.

**22.** Verfahren für die Herstellung einer Carvedilol enthaltenden pharmazeutischen Zusammensetzung in einer Form von geschichteten Pellets gemäß Anspruch 21, **dadurch gekennzeichnet, dass**

a) 5 - 50 Gew.-% einer festen organischen Säure auf Basis des Gesamtgewichts der Pellets und gegebenenfalls 5 - 50 Gew.-% eines oder mehrerer Hilfsmittel auf Basis des Gesamtgewichts der Pellets in einer bekannten Weise granuliert werden, die erhaltenen Pelletkerne (1) getrocknet werden und
b) mit 0,5 - 10 Gew.-% eines Beschichtungspolymers in Form eines enterischen Films auf Basis des Gesamtgewichts der Pellets und gegebenenfalls einem oder mehreren Hilfsmitteln, vorzugsweise Weichmachern und Antihaftmitteln, in einer üblichen Weise beschichtet und gegebenenfalls getrocknet werden,
c) die Oberfläche der enterischen Schicht (2) mit einer Mischung von 5 - 30 Gew.-% Carvedilol auf Basis des Gesamtgewichts der Pellets und 5 - 30 Gew.-% eines wasserlöslichen Bindemittels und gegebenenfalls unter Verwendung anderer Hilfsmittel in einer üblichen Weise beschichtet wird, die erhaltenen beschichteten Pellets gegebenenfalls getrocknet werden, danach
d) die Carvedilol enthaltende Schicht (3) mit einer die Auflösung regulierenden Schicht (4) in einer üblichen Weise mit einer Mischung von 0,5 - 10 Gew.-% eines enterischen Beschichtungspolymers und 0,5 - 10 Gew.-% eines wasserlöslichen Polymers auf Basis des Gesamtgewichts der Pellets beschichtet wird.

**23.** Verfahren für die Herstellung einer Carvedilol enthaltenden pharmazeutischen Zusammensetzung in einer Form von geschichteten Pellets gemäß Anspruch 21, **dadurch gekennzeichnet, dass**

a) 10 - 40 Gew.-% einer festen organischen Säure auf Basis des Gesamtgewichts der Pellets und gegebenenfalls 10 - 40 Gew.-% von einem oder mehreren Arzneimittelträgem auf Basis des Gesamtgewichts der Pellets in einer bekannten Weise granuliert werden, die erhaltenen Pelletkerne (1) getrocknet werden und
b) mit 1 - 5 Gew.-% eines Beschichtungspolymers in Form eines enterischen Films auf Basis des Gesamtgewichts der Pellets und gegebenenfalls einem oder mehreren Hilfsmitteln, vorzugsweise Weichmachern und Antihaftmitteln, in einer üblichen Weise beschichtet und gegebenenfalls getrocknet werden,
c) die Oberfläche der enterischen Schicht (2) mit einer Mischung von 10 - 20 Gew.-% Carvedilol auf Basis des Gesamtgewichts der Pellets und 10 - 20 Gew.-% eines wasserlöslichen Bindemittels und gegebenenfalls unter Verwendung anderer Hilfsmittel in einer üblichen Weise beschichtet wird, die erhaltenen beschichteten Pellets

gegebenenfalls getrocknet werden, danach
d) die Carvedilol enthaltende Schicht (3) mit einer die Auflösung regulierenden Schicht (4) in einer üblichen Weise mit einer Mischung von 1 - 5 Gew.-% eines enterischen Beschichtungspolymers und 1 - 5 Gew.-% eines wasserlöslichen Polymers auf Basis des Gesamtgewichts der Pellets beschichtet wird.

**24.** Verfahren für die Herstellung einer Carvedilol enthaltenden pharmazeutischen Zusammensetzung in einer Form von geschichteten Pellets gemäß Anspruch 21, **dadurch gekennzeichnet, dass**

a) 20 - 40 Gew.-% einer festen organischen Säure auf Basis des Gesamtgewichts der Pellets und gegebenenfalls 20 - 40 Gew.-% eines oder mehrerer Hilfsmittel auf Basis des Gesamtgewichts der Pellets in einer bekannten Weise granuliert werden, die erhaltenen Pelletkerne (1) getrocknet werden und
b) mit 1 - 3 Gew.-% eines Beschichtungspolymers in Form eines enterischen Films auf Basis des Gesamtgewichts der Pellets und gegebenenfalls einem oder mehreren Hilfsmitteln, vorzugsweise Weichmachern und Antihaftmitteln, in einer üblichen Weise beschichtet und gegebenenfalls getrocknet werden,
c) die Oberfläche der enterischen Schicht (2) mit einer Mischung von 10 - 15 Gew.-% Carvedilol auf Basis des Gesamtgewichts der Pellets und 10 - 15 Gew.-% eines wasserlöslichen Bindemittels und gegebenenfalls unter Verwendung anderer Hilfsmittel in einer bekannten Weise beschichtet wird, die erhaltenen beschichteten Pellets gegebenenfalls getrocknet werden, danach
d) die Carvedilol enthaltende Schicht (3) mit einer die Auflösung regulierenden Schicht (4) in einer bekannten Weise mit einer Mischung von 1 - 3 Gew.-% eines enterischen Beschichtungspolymers und 1 - 3 Gew.-% eines wasserlöslichen Polymers auf Basis des Gesamtgewichts der Pellets beschichtet wird.

**25.** Verfahren gemäß einem beliebigen der Ansprüche 21 - 24, **dadurch gekennzeichnet, dass**

a) die organische Säure und die Hilfsmittel homogenisiert werden, danach die erhaltene homogenisierte Mischung mit Wasser granuliert wird, welches gegebenenfalls weitere Hilfsmittel enthalten kann, und die erhaltenen Granalien getrocknet werden,
b) die erhaltenen Pelletkerne (1) durch Sprühen einer Dispersion einer Mischung einer Beschichtungspolymerdispersion in Form eines enterischen Films und gegebenenfalls von anderen Hilfsmitteln auf die Kerne beschichtet werden, die erhaltenen beschichteten Pellets bei Bedarf getrocknet werden,
c) die enterischen beschichteten Pellets mit einer Suspension einer Mischung von Carvedilol und einem wasserlöslichen Bindemittel besprüht werden, gegebenenfalls andere Hilfsmittel auf die enterischen beschichteten Pellets aufgebracht werden, oder
durch anschließende Aufbringung des pulverförmigen Carvedilols und der Dispersion des wasserlöslichen Bindemittels und gegebenenfalls die erhaltenen beschichteten Pellets getrocknet werden, danach
d) eine Mischung von enterischem Beschichtungspolymer, wasserlöslichem Polymer und gegebenenfalls anderen Hilfsmitteln in der Form einer Lösung oder Dispersion, die Wasser oder organische Lösungsmittel enthält, vorzugsweise in der Form einer alkoholischen Lösung oder einer Dispersion auf die Carvedilol enthaltende Schicht gesprüht wird.

**26.** Verfahren gemäß einem beliebigen der Ansprüche 21 - 25, **dadurch gekennzeichnet, dass** die für die Herstellung der Pelletkerne (1) verwendete organische Säure physiologisch inerte, feste, organische Säuren mit Ausnahme von Fumarsäure sind, vorzugsweise gesättigte und ungesättigte Di- und Tricarbonsäuren mit 4 - 5 Kohlenstoffatomen, stärker bevorzugt Citronensäure, Weinsäure, Äpfelsäure, Maleinsäure, Bernsteinsäure, und am meisten bevorzugt Bernsteinsäure.

**27.** Verfahren gemäß einem beliebigen der Ansprüche 21 - 26, wobei die verwendeten Hilfsmittel für die Herstellung der Pelletkerne (1) in der pharmazeutischen Industrie eingesetzt werden, vorzugsweise Lactose, Stärke, pulverförmige Cellulose, mikrokristalline Cellulose, kolloidales Siliciumdioxid, Dimethylpolysiloxan oder Mischungen davon, stärker bevorzugt mikrokristalline Cellulose, kolloidales Siliciumdioxid, Dimethylpolysiloxan oder Mischungen davon.

**28.** Verfahren gemäß einem beliebigen der Ansprüche 21 - 27, **dadurch gekennzeichnet, dass** die Oberfläche des Pelletkerns (1) mit Beschichtungspolymer oder -polymeren in Form eines enterischen Films, vorzugsweise Methacrylsäure-Ethylacrylat-Copolymer, Methacrylsäure-Methylmethacrylat-Copolymer, Celluloseacetatphthalat, Hydroxypropylmethylcellulosephthalat, Hydroxypropylmethylcelluloseacetatsuccinat oder Polyvinylacetatphthalat, stärker bevorzugt Methacrylsäure-Ethylacrylat-Copolymer beschichtet wird.

**29.** Verfahren gemäß Anspruch 28, **dadurch gekennzeichnet, dass** andere Hilfsmittel, gegebenenfalls Weichmacher

und Antihaftverbindungen, ebenfalls in dem Aufbringungsverfahren der enterischen Überzugsschicht (2) auf den Pelletkern (1) verwendet werden.

**30.** Verfahren gemäß Anspruch 29, **dadurch gekennzeichnet, dass** Propylenglykol, Triethylcitrat, Polyethylenglykol oder eine Mischung davon als Weichmacher in dem Aufbringungsverfahren der enterischen Überzugsschicht (2) auf den Pelletkern (1) verwendet werden.

**31.** Verfahren gemäß Anspruch 29, **dadurch gekennzeichnet, dass** Talk, Dimethylpolysiloxan oder eine Mischung davon als Antihaftmittel in dem Aufbringungsverfahren der enterischen Überzugsschicht (2) auf den Pelletkern (1) verwendet werden.

**32.** Verfahren gemäß einem beliebigen der Ansprüche 21 - 31, **dadurch gekennzeichnet, dass** die Carvedilol enthaltende Schicht (3) hergestellt wird unter Verwendung von Hydroxypropylcellulose, Hydroxypropylmethylcellulose, Hydroxyethylcellulose, Polyvinylpyrrolidon, Vinylpyrrolidon/Vinylacetat-Copolymer, Polyvinylalkohol, Polyethylenglykol, Polyvinylalkohol-Polyethylenglykol-Pfropfcopolymer oder Mischungen davon, vorzugsweise einer Mischung von Hydroxypropylcellulose und Polyethylenglykol als Beschichtungsmittel in Form eines wasserlöslichen Films.

**33.** Verfahren gemäß Anspruch 32, **dadurch gekennzeichnet, dass** weitere Hilfsmittel verwendet werden.

**34.** Verfahren gemäß einem beliebigen der Ansprüche 21 - 33, **dadurch gekennzeichnet, dass** die Carvedilol enthaltende Schicht (3) durch die nachfolgende Aufbringung von pulverförmigem Carvedilol und des wasserlöslichen Bindemittels oder durch Sprühen einer Suspension, die eine Mischung des Carvedilols und der wässrigen Mischung des wasserlöslichen Bindemittels enthält, hergestellt wird.

**35.** Verfahren gemäß einem beliebigen der Ansprüche 21 - 34, **dadurch gekennzeichnet, dass** Hydroxypropylcellulose, Hydroxypropylmethylcellulose, Hydroxyethylcellulose, Polyvinylpyrrolidon, Vinylpyrrolidon/Vinylacetat-Copolymer, Polyvinylalkohol, Polyethylenglykol, Polyvinylalkohol-Polyethylenglykol-Pfropfcopolymer oder Mischungen davon, vorzugsweise Hydroxypropylcellulose, als Beschichtungsverbindungen in Form eines wasserlöslichen Films für die Herstellung der äußeren Schicht (4) auf der Oberfläche der Carvedilol enthaltenden Schicht (3) verwendet werden.

**36.** Verfahren gemäß einem beliebigen der Ansprüche 21 - 34, **dadurch gekennzeichnet, dass** Methacrylsäure-Ethylacrylat-Copolymer, Methacrylsäure-Methylmethacrylat-Copolymer, Celluloseacetatphthalat, Hydroxymethylcellulosephthalat, Hydroxypropylmethylcelluloseacetatsuccinat, oder Polyvinylacetatphthalat oder Mischungen davon, vorzugsweise Methacrylsäure-Ethylacrylat-Copolymer als Beschichtungsverbindung in Form eines wasserlöslichen Films für die Herstellung der äußeren Schicht (4) auf der Oberfläche der Carvedilol enthaltenden Schicht (3) verwendet werden.

**37.** Verfahren für die Herstellung einer Carvedilol enthaltenden pharmazeutischen Zusammensetzung in einer Form von mehrschichtigen Pellets gemäß Anspruch 21**, dadurch gekennzeichnet, dass**

a) 25 - 35 Gew.-% Bernsteinsäure und gegebenenfalls 25 - 35 Gew.-% mikrokristalline Cellulose mit 0,4 - 1 Gew.-% Dimethylpolysiloxan enthaltendem Wasser auf Basis des Gesamtgewichts der Pellets granuliert werden, die erhaltenen Pelletkerne (1) getrocknet werden und
b) mit 1 - 5 Gew.-% Methacrylsäure-Ethylacrylat-Copolymer, gegebenenfalls mit einer Mischung von 0,1 - 0,35 Gew.-% Propylenglykol und 0,1 - 0,15 Gew.-% Antihaftmittel auf Basis des Gesamtgewichts der Pellets durch Sprühen auf die Oberfläche der Kerne (1) beschichtet werden, die erhaltenen beschichteten Pellets gegebenenfalls getrocknet werden, danach
c) auf die Oberfläche der enterischen Schicht (2) eine weitere Schicht durch Besprühen der beschichteten Pellets mit einer Mischung von 10 - 15 Gew.-% Carvedilol auf Basis des Gewichts und 10 - 15 Gew.-% einer Mischung von Hydroxypropyl-methylcellulose, Polyethylenglykol und gegebenenfalls weiteren Hilfsmitteln aufgebracht wird,
oder
die Schicht durch die nachfolgende Aufbringung von 5 - 30 Gew.-% pulverförmigem Carvedilol und 5 - 30 Gew.-% der Mischung von Hydroxymethylcellulosepolyethylenglykol und gegebenenfalls weiteren Hilfsmitteln hergestellt wird, die erhaltenen Pellets gegebenenfalls getrocknet werden, danach
d) die erhaltene Carvedilol enthaltende Schicht (3) durch Besprühen mit einer alkoholischen Mischung von 1 - 3 Gew.-% Methacrylsäure-Ethylacrylat-Copolymer und 1 - 3 Gew.-% Hydroxypropylcellulose auf Basis des

Gesamtgewichts des Pellets beschichtet wird.

38. Verfahren für die Herstellung einer festen Dosierungsform zur kontrollierten Freisetzung gemäß einem beliebigen der Ansprüche 1 - 14, **dadurch gekennzeichnet, dass** die Pellets gegebenenfalls mit Hilfsmitteln vermischt werden, die allgemein in der pharmazeutischen Industrie eingesetzt werden, und dann zu der galenischen Form umgewandelt werden.

39. Verfahren für die Herstellung einer festen Dosierungsform zur kontrollierten Freisetzung gemäß Anspruch 38, wobei 40 - 90 Gew.-%, vorzugsweise 60 - 90 Gew.-%, stärker bevorzugt 80 - 90 Gew.-% Pellets gemäß einem beliebigen der Ansprüche 1 - 14, 10 - 50 Gew.-%, vorzugsweise 10 - 40 Gew.-%, stärker bevorzugt 10 - 20 Gew.-% des Füllstoffs, gegebenenfalls 1 - 10 Gew.-%, vorzugsweise 2 - 6 Gew.-% des Sprengmittels, 2 - 10 Gew.-%, vorzugsweise 3 - 8 Gew.-%, stärker bevorzugt 0,1 - 5 Gew.-% des Bindemittels und gegebenenfalls 0,1 - 1 Gew.-% Gleitmittel gemischt und zu Tabletten gepresst werden.

40. Verfahren für die Herstellung einer festen Dosierungsform zur kontrollierten Freisetzung gemäß Anspruch 38, wobei 40 - 100 Gew.-%, vorzugsweise 60 - 100 Gew.-%, stärker bevorzugt 80 - 100 Gew.-% der Pellets gemäß einem beliebigen der Ansprüche 1 - 14, gegebenenfalls 0,1 - 60 Gew.-%, vorzugsweise 0,1 - 20 Gew.-%, stärker bevorzugt 0,1 - 10 Gew.-% des Füllstoffs, gegebenenfalls 0,1 - 20 Gew.-%, vorzugsweise 0,1 - 10 Gew.-%, stärker bevorzugt 0,1 - 5 Gew.-% des Sprengmittels, 2 - 10 Gew.-%, vorzugsweise 3 - 8 Gew.-%, stärker bevorzugt 0,1 - 5 Gew.-% des Bindemittels und gegebenenfalls 0,1 - 2 Gew.-%, vorzugsweise 0,1 - 1 Gew.-% des Gleitmittels gemischt und in Kapseln gefüllt werden.

41. Verfahren für die Herstellung von Kapseln gemäß Anspruch 40, **dadurch gekennzeichnet, dass** harte Gelatinekapseln verwendet werden.

42. Verfahren für die Herstellung von Dosierungsformen gemäß einem beliebigen der Ansprüche 38 - 41, **dadurch gekennzeichnet, dass** die allgemein annehmbaren Arzneimittelträger, vorzugsweise Lactose, Stärke, pulverförmige Cellulose, mikrokristalline Cellulose, kolloidales Siliciumdioxid, Dimethylpolysiloxan oder Mischungen davon, am meisten bevorzugt mikrokristalline Cellulose, kolloidales Siliciumdioxid, Dimethylpolysiloxan oder Mischungen davon verwendet werden, beliebiges pharmazeutisch annehmbares Bindemittel, vorzugsweise Polyvinylpyrrolidon, Hydroxypropylcellulose verwendet werden, beliebige der pharmazeutisch akzeptierten Sprengmittel, vorzugsweise Natriumcarboxymethylcellulose, vernetztes Polyvinylpyrrolidon, Natriumcarboxymethylstärke oder niedrig substituierte Hydroxypropylcellulose, stärker bevorzugt Crospovidon, verwendet werden, beliebige der pharmazeutisch annehmbaren Gleitmittel, vorzugsweise Calcium- und Magnesiumstearat, Glycerylbehanat, Stearinsäuretalk oder hydrierte pflanzliche Öle verwendet werden können.

## Revendications

1. Composition pharmaceutique comprenant du carvédilol selon la formule

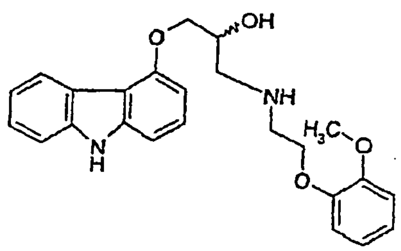

(I)

sous la forme d'une pilule stratifiée, dans laquelle ladite pilule contient un coeur (1) contenant un acide organique solide, une couche gastro-résistante (2) sur la surface du coeur, une couche (3) contenant du carvédilol et un agent liant hydrosoluble sur la surface de la couche gastro-résistante (2), et une couche externe contrôlant la dissolution (4) contenant un mélange de polymère hydrosoluble et d'un polymère gastro-résistant, sur la surface de la couche contenant l'ingrédient actif.

2. Composition pharmaceutique contenant du carvédilol sous la forme d'une pilule stratifiée selon la revendication 1, dans laquelle la pilule a un coeur (1) contenant 5 à 50 % en poids d'acide organique solide par rapport au poids total de la pilule, et facultativement 5 à 50 % en poids d'un ou plusieurs agents auxiliaires, une couche gastro-résistante (2) sur la surface du coeur contenant 0,5 à 10 % en poids de polymère gastro-résistant par rapport au poids total de la pilule, et facultativement d'autres agents auxiliaires, une autre couche (3) sur la surface de la couche gastro-résistante contenant 5 à 30 % en poids de carvédilol par rapport au poids total de la pilule, et 5 à 30 % en poids de liant hydrosoluble par rapport au poids total de la pilule et facultativement d'autres agents auxiliaires, une autre couche contrôlant la dissolution (4) sur la surface de la couche contenant l'ingrédient actif, contenant un mélange de 0,5 à 10 % en poids de polymère hydrosoluble par rapport au poids total de la pilule et de 0,5 à 10 % en poids de polymère gastro-résistant par rapport au poids total de la pilule et facultativement d'autres agents auxiliaires.

3. Composition pharmaceutique contenant du carvédilol sous la forme d'une pilule stratifiée selon la revendication 1, dans laquelle la pilule a un coeur (1) contenant 10 à 40 % en poids d'acide organique solide par rapport au poids total de la pilule et facultativement 10 à 40 % en poids d'un ou plusieurs agents auxiliaires, une couche gastro-résistante (2) sur la surface du coeur contenant 1 à 5 % en poids de polymère gastro-résistant par rapport au poids total de la pilule et facultativement d'autres agents auxiliaires, sur la surface de la couche gastro-résistante une autre couche (3) contenant 10 à 20 % en poids de carvédilol par rapport au poids total de la pilule et 10 à 20 % en poids de liant hydrosoluble par rapport au poids de la pilule totale et facultativement d'autres agents auxiliaires, sur la surface de la couche ayant l'ingrédient actif une autre couche contrôlant la dissolution (4) contenant un mélange de 1 à 5 % en poids de polymère hydrosoluble par rapport au poids total de la pilule et de 1 à 5 % en poids de polymère gastro-résistant par rapport au poids total de la pilule et facultativement d'autres agents auxiliaires.

4. Composition pharmaceutique contenant du carvédilol sous la forme d'une pilule stratifiée selon la revendication 1, dans laquelle la pilule a un coeur (1) contenant 20 à 40 % en poids d'acide organique solide par rapport au poids total de la pilule et facultativement 20 à 40 % en poids d'un ou plusieurs agents auxiliaires, une couche gastro-résistante (2) sur la surface du coeur contenant 1 à 3 % en poids de polymère gastro-résistant par rapport au poids total de la pilule et facultativement d'autres agents auxiliaires, sur la surface de la couche gastro-résistante une autre couche (3) contenant 10 à 15 % en poids de carvédilol par rapport au poids de la pilule et 10 à 15 % en poids de liant hydrosoluble par rapport au poids total de la pilule et facultativement d'autres agents auxiliaires, sur la surface de la couche ayant l'ingrédient actif, une autre couche contrôlant la dissolution (4) contenant un mélange de 1 à 3 % en poids de polymère hydrosoluble par rapport au poids total de la pilule et de 1 à 3 % en poids de polymère gastro-résistant par rapport au poids total de la pilule et facultativement d'autres agents auxiliaires.

5. Composition pharmaceutique comprenant du carvédilol sous la forme d'une pilule stratifiée selon l'une quelconque des revendications 1 à 4, **caractérisée en ce que** le coeur contient un acide organique solide physiologiquement inerte, de préférence un acide di ou tricarboxylique, saturé ou insaturé, ayant 4 à 5 atomes de carbone, sauf l'acide fumarique, de préférence encore l'acide citrique, l'acide tartrique, l'acide malique, l'acide maléique, l'acide succinique et de toute préférence l'acide succinique.

6. Composition pharmaceutique contenant du carvédilol sous la forme d'une pilule stratifiée selon l'une quelconque des revendications 1 à 4, dans laquelle les agents auxiliaires utilisés pour la préparation du coeur (1) de la pilule sont généralement acceptés pour la préparation de pilules dans l'industrie pharmaceutique, de préférence le lactose, l'amidon, la cellulose en poudre, la cellulose microcristalline, le dioxyde de silicium colloïdal, un diméthylpolysiloxane ou un mélange de ceux-ci, de toute préférence la cellulose microcristalline et facultativement un diméthylpolysiloxane.

7. Composition pharmaceutique contenant du carvédilol sous la forme d'une pilule stratifiée selon l'une quelconque des revendications 1 à 6, dans laquelle la couche gastro-résistante (2) sur la surface du coeur (1) de la pilule contient un polymère d'enrobage gastro-résistant, de préférence un copolymère acide méthacrylique-acrylate d'éthyle, un copolymère acide méthacrylique-méthacrylate de méthyle, l'acétophtalate de cellulose, l'acétosuccinate d'hydroxypropylméthylcellulose ou un acétophtalate de polyvinyle, de préférence encore un copolymère acide méthacrylique-acrylate d'éthyle.

8. Composition pharmaceutique contenant du carvédilol sous la forme d'une pilule stratifiée selon l'une quelconque des revendications 1 à 6, dans laquelle la couche gastro-résistante (2) sur la surface du coeur (1) de la pilule contient d'autres agents auxiliaires tels que des émollients ou facultativement des agents antiadhésifs.

9. Composition pharmaceutique contenant du carvédilol sous la forme d'une pilule stratifiée selon la revendication 8,

dans laquelle la couche gastro-résistante (2) contient du propylèneglycol, du citrate de triéthyle, un polyéthylèneglycol ou des mélanges de ceux-ci en tant qu'émollient.

**10.** Composition pharmaceutique contenant du carvédilol sous la forme d'une pilule stratifiée selon l'une quelconque des revendications 8 ou 9, dans laquelle la couche gastro-résistante (2) contient du talc, un diméthylpolysiloxane ou des mélanges de ceux-ci en tant qu'agents antiadhésifs.

**11.** Composition pharmaceutique contenant du carvédilol sous la forme d'une pilule stratifiée selon l'une quelconque des revendications 1 à 10, dans laquelle la couche comprenant du carvédilol (3) contient de l'hydroxypropylcellulose, de l'hydroxypropylméthylcellulose, de l'hydroxyéthylcellulose, une polyvinylpyrrolidone, un copolymère vinylpyrrolidone/acétate de vinyle, un alcool polyvinylique, un polyéthylèneglycol, un copolymère greffé alcool polyvinylique-polyéthylèneglycol ou des mélanges de ceux-ci, de préférence un mélange d'hydroxypropylméthylcellulose et de polyéthylèneglycol, en tant qu'agents liants hydrosolubles.

**12.** Composition pharmaceutique contenant du carvédilol sous la forme d'une pilule stratifiée selon l'une quelconque des revendications 1 à 11, dans laquelle la couche (4) sur la surface de la couche comprenant du carvédilol (3) contient de l'hydroxypropylcellulose, de l'hydroxypropylméthylcellulose, de l'hydroxyéthylcellulose, une polyvinylpyrrolidone, un copolymère vinylpyrrolidone/acétate de vinyle, un alcool polyvinylique, un polyéthylèneglycol, un copolymère greffé alcool polyvinylique-polyéthylèneglycol ou des mélanges de ceux-ci, de préférence de l'hydroxypropylcellulose, en tant qu'agent d'enrobage pelliculaire hydrosoluble.

**13.** Composition pharmaceutique contenant du carvédilol sous la forme d'une pilule stratifiée selon l'une quelconque des revendications 1 à 12, dans laquelle la couche (4) sur la surface de la couche comprenant du carvédilol (3) contient un copolymère acide méthacrylique-acrylate d'éthyle, un copolymère acide méthacrylique-méthacrylate de méthyle, de l'acétophtalate de cellulose, du phtalate d'hydroxyméthylcellulose, de l'acétosuccinate d'hydroxypropylméthylcellulose ou de l'acétophtalate de polyvinyle, ou des mélanges de ceux-ci, de préférence un copolymère acide méthacrylique-acrylate d'éthyle, en tant qu'agent d'enrobage pelliculaire gastro-résistant.

**14.** Composition pharmaceutique contenant du carvédilol sous la forme d'une pilule stratifiée selon la revendication 1, dans laquelle la pilule a un coeur (1) contenant 25 à 35 % en poids d'acide succinique par rapport au poids total de la pilule et 25 à 35 % en poids de cellulose microcristalline et 0,4 à 1 % en poids de diméthylpolysiloxane, une couche gastro-résistante (2) sur la surface du coeur contenant 1 à 5 % en poids de copolymère acide méthacrylique-acrylate d'éthyle et facultativement en outre 0,1 à 0,35 % en poids de propylèneglycol, sur la surface de la couche gastro-résistante une autre couche (3) contenant 10 à 15 % en poids de carvédilol par rapport au poids total de la pilule et 10 à 15 % en poids d'un mélange d'hydroxypropylméthylcellulose et de polyéthylèneglycol par rapport au poids total de la pilule, sur la surface de la couche contenant l'ingrédient actif, et une autre couche (4) contenant un mélange de 1 à 3 % en poids d'hydroxypropylcellulose par rapport au poids total de la pilule et de 1 à 3 % en poids de copolymère acide méthacrylique-acrylate d'éthyle par rapport au poids total de la pilule.

**15.** Forme pharmaceutique posologique solide à libération lente, qui contient des pilules selon l'une quelconque des revendications 1 à 14 et facultativement d'autres agents auxiliaires utilisés dans l'industrie pharmaceutique.

**16.** Forme pharmaceutique posologique solide à libération lente selon la revendication 15, **caractérisée en ce que** la forme posologique est un comprimé ou une capsule.

**17.** Forme posologique solide selon la revendication 16, **caractérisée en ce que** les comprimés contiennent 40 à 90 % en poids, de préférence 60 à 90 % en poids, de préférence encore 80 à 90 % en poids de pilules, 10 à 50 % en poids, de préférence 10 à 40 % en poids, de préférence encore 10 à 20 % en poids de charge, 1 à 10 % en poids, de préférence 2 à 6 % en poids, de préférence encore 3 à 5 % en poids d'agent désintégrant, 2 à 10 % en poids, de préférence 3 à 8 % en poids, de préférence encore 0,1 à 5 % en poids d'agent liant, 0,1 à 1 % en poids, de préférence 0,1 à 1 % en poids de lubrifiant.

**18.** Forme posologique solide selon la revendication 16, **caractérisée en ce que** les capsules contiennent 40 à 100 % en poids, de préférence 60 à 100 % en poids, de préférence encore 80 à 100 % en poids de pilules, facultativement 0,1 à 60 % en poids, de préférence 0,1 à 40 % en poids, de préférence encore 0,1 à 20 % en poids de charge, 0,1 à 10 % en poids, de préférence 0,1 à 5 % en poids d'agent désintégrant, 0,1 à 2 % en poids, de préférence 0,1 à 1 % en poids de lubrifiant.

**19.** Capsules selon la revendication 18, **caractérisées en ce que** des capsules en gélatine dure sont utilisées.

**20.** Forme pharmaceutique posologique solide à libération lente contenant des pilules selon l'une quelconque des revendications 15 à 19, **caractérisée en ce qu'**une charge pharmaceutiquement acceptable, de préférence le lactose, l'amidon, la cellulose en poudre, la cellulose microcristalline, le dioxyde de silicium colloïdal, un diméthylpolysiloxane ou des mélanges de ceux-ci, de préférence encore la cellulose microcristalline, le dioxyde de silicium colloïdal, un diméthylpolysiloxane ou des mélanges de ceux-ci, est utilisée en tant que charge, un agent liant pharmaceutiquement acceptable est de préférence la carboxyméthylcellulose ou l'hydroxypropylcellulose peu substituée, un agent désintégrant pharmaceutiquement accepté est de préférence la carboxyméthylcellulose sodique, une polyvinylpyrrolidone réticulée est un amidon carboxyméthylé sodique, ou l'hydroxypropylcellulose peu substituée, de préférence encore la crospovidone, des lubrifiants pharmaceutiquement acceptables sont de préférence le stéarate de calcium et de magnésium, le béhénate de glycéryle, l'acide stéarique, le talc ou les huiles végétales hydrogénées.

**21.** Procédé pour la préparation d'une composition pharmaceutique contenant du carvédilol sous la forme de pilules stratifiées, **caractérisé en ce que**

a) un acide organique solide et facultativement un ou plusieurs excipients sont granulés d'une manière connue, le coeur (1) de pilule obtenu
b) est enrobé avec un polymère d'enrobage gastro-résistant d'une manière connue, les pilules enrobées obtenues sont facultativement séchées,
c) la surface de la couche gastro-résistante (2) est enrobée avec un mélange de carvédilol et d'un agent liant hydrosoluble facultativement en utilisant d'autres agents auxiliaires d'une manière connue, facultativement séchée,
d) les pilules enrobées obtenues sont enrobées d'une manière connue avec un mélange d'un polymère hydrosoluble et d'un polymère d'enrobage gastro-résistant.

**22.** Procédé pour la préparation d'une composition pharmaceutique contenant du carvédilol sous la forme de pilules stratifiées selon la revendication 21, **caractérisé en ce que**

a) 5 à 50 % en poids d'acide organique solide par rapport au poids total des pilules et facultativement 5 à 50 % en poids d'un ou plusieurs agents auxiliaires par rapport au poids total des pilules sont granulés d'une manière connue, les coeurs (1) de pilules obtenus sont séchés, et
b) enrobés avec 0,5 à 10 % en poids de polymère d'enrobage pelliculaire gastro-résistant par rapport au poids total des pilules et facultativement un ou plusieurs agents auxiliaires, de préférence des émollients et des agents antiadhésifs, d'une manière habituelle et facultativement séchés,
c) la surface de la couche gastro-résistante (2) est enrobée avec un mélange de 5 à 30 % en poids de carvédilol par rapport au poids total des pilules et de 5 à 30 % en poids d'agent liant hydrosoluble et en utilisant facultativement d'autres agents auxiliaires d'une manière habituelle, les pilules enrobées obtenues sont facultativement séchées, puis
d) la couche contenant du carvédilol (3) est enrobée avec une couche contrôlant la dissolution (4) d'une manière habituelle avec un mélange de 0,5 à 10 % en poids de polymère d'enrobage gastro-résistant et de 0,5 à 10 % en poids de polymère hydrosoluble par rapport au poids total des pilules.

**23.** Procédé pour la préparation d'une composition pharmaceutique contenant du carvédilol sous la forme de pilules stratifiées selon la revendication 21, **caractérisé en ce que**

a) 10 à 40 % en poids d'acide organique solide par rapport au poids total des pilules et facultativement 10 à 40 % en poids d'un ou plusieurs excipients par rapport au poids total des pilules sont granulés d'une manière connue, les coeurs (1) de pilules obtenus sont séchés, et
b) enrobés avec 1 à 5 % en poids de polymère d'enrobage pelliculaire gastro-résistant par rapport au poids total des pilules et facultativement un ou plusieurs agents auxiliaires, de préférence des émollients et des agents antiadhésifs, d'une manière habituelle et facultativement séchés,
c) la surface de la couche gastro-résistante (2) est enrobée avec un mélange de 10 à 20 % en poids de carvédilol par rapport au poids total des pilules et de 10 à 20 % en poids d'agent liant hydrosoluble et en utilisant facultativement d'autres agents auxiliaires d'une manière habituelle, les pilules enrobées obtenues sont facultativement séchées, puis
d) la couche contenant du carvédilol (3) est enrobée avec une couche contrôlant la dissolution (4) d'une manière

habituelle avec un mélange de 1 à 5 % en poids de polymère d'enrobage gastro-résistant et de 1 à 5 % en poids de polymère hydrosoluble par rapport au poids total des pilules.

**24.** Procédé pour la préparation d'une composition pharmaceutique contenant du carvédilol sous la forme de pilules stratifiées selon la revendication 21, **caractérisé en ce que**

a) 20 à 40 % en poids d'acide organique solide par rapport au poids total des pilules et facultativement 20 à 40 % en poids d'un ou plusieurs agents auxiliaires par rapport au poids total des pilules sont granulés d'une manière connue, les coeurs (1) de pilules obtenus sont séchés, et
b) enrobés avec 1 à 3 % en poids de polymère d'enrobage pelliculaire gastro-résistant par rapport au poids total des pilules et facultativement un ou plusieurs agents auxiliaires, de préférence des émollients et des agents antiadhésifs, d'une manière connue et facultativement séchés,
c) la surface de la couche gastro-résistante (2) est enrobée avec un mélange de 10 à 15 % en poids de carvédilol par rapport au poids total des pilules et de 10 à 15 % en poids d'agent liant hydrosoluble et en utilisant facultativement d'autres agents auxiliaires d'une manière connue, les pilules enrobées obtenues sont facultativement séchées, puis
d) la couche contenant du carvédilol (3) est enrobée avec une couche contrôlant la dissolution (4) d'une manière connue avec un mélange de 1 à 3 % en poids de polymère d'enrobage gastro-résistant et de 1 à 3 % en poids de polymère hydrosoluble par rapport au poids total des pilules.

**25.** Procédé selon l'une quelconque des revendications 21 à 24, **caractérisé en ce que**

a) l'acide organique et les agents auxiliaires sont homogénéisés, puis le mélange homogénéisé obtenu est granulé avec de l'eau qui peut facultativement contenir d'autres agents auxiliaires, et les granules obtenus sont séchés,
b) les coeurs (1) de pilules obtenus sont enrobés en pulvérisant une dispersion d'un mélange de dispersion de polymère d'enrobage pelliculaire gastro-résistant et facultativement d'autres agents auxiliaires sur les coeurs, les pilules enrobées obtenues sont séchées si nécessaire,
c) les pilules enrobées gastro-résistantes sont pulvérisées avec une suspension d'un mélange de carvédilol et d'un agent liant hydrosoluble, facultativement d'autres agents auxiliaires sont appliqués sur les pilules enrobées gastro-résistantes ou, par application subséquente du carvédilol en poudre et de la dispersion de l'agent liant hydrosoluble, et facultativement les pilules enrobées obtenues sont séchées, puis
d) un mélange de polymère d'enrobage gastro-résistant, de polymère hydrosoluble et facultativement d'autres agents auxiliaires sont pulvérisés sous la forme d'une solution ou d'une dispersion contenant de l'eau ou des solvants organiques, de préférence sous la forme d'une solution ou dispersion alcoolique sur la couche contenant du carvédilol.

**26.** Procédé selon l'une quelconque des revendications 21 à 25, **caractérisé en ce que** l'acide organique utilisé pour la préparation des coeurs (1) de pilules est un acide organique solide physiologiquement inerte, sauf l'acide fumarique, de préférence des acides di et tricarboxyliques, saturés et insaturés, ayant 4 à 5 atomes de carbone, de préférence encore l'acide citrique, l'acide tartrique, l'acide malique, l'acide maléique, l'acide succinique et de toute préférence l'acide succinique.

**27.** Procédé selon l'une quelconque des revendications 21 à 26, dans lequel les agents auxiliaires utilisés pour la préparation des coeurs (1) de pilules sont utilisés dans l'industrie pharmaceutique, de préférence le lactose, l'amidon, la cellulose en poudre, la cellulose microcristalline, le dioxyde de silicium colloïdal, un diméthylpolysiloxane ou des mélanges de ceux-ci, de préférence encore la cellulose microcristalline, le dioxyde de silicium colloïdal, un diméthylpolysiloxane ou des mélanges de ceux-ci.

**28.** Procédé selon l'une quelconque des revendications 21 à 27, **caractérisé en ce que** la surface du coeur (1) de la pilule est enrobée avec un ou des polymères d'enrobage pelliculaire gastro-résistants, de préférence un copolymère acide méthacrylique-acrylate d'éthyle, un copolymère acide méthacrylique-méthacrylate de méthyle, l'acétophtalate de cellulose, le phtalate d'hydroxypropylméthylcellulose, l'acétosuccinate d'hydroxypropylméthylcellulose ou un acétophtalate de polyvinyle, de préférence encore un copolymère acide méthacrylique-acrylate d'éthyle.

**29.** Procédé selon la revendication 28, **caractérisé en ce que** d'autres agents auxiliaires, facultativement des émollients et des composés antiadhésifs, sont aussi utilisés lors du traitement d'application de la couche d'enrobage gastro-résistante (2) sur le coeur (1) de pilule.

**30.** Procédé selon la revendication 29, **caractérisé en ce que** du propylèneglycol, du citrate de triéthyle, un polyéthylèneglycol ou un mélange de ceux-ci sont utilisés en tant qu'émollient lors du traitement d'application de la couche d'enrobage gastro-résistante (2) sur le coeur (1) de pilule.

**31.** Procédé selon la revendication 29, **caractérisé en ce que** du talc, un diméthylpolysiloxane ou un mélange de ceux-ci sont utilisés en tant qu'agent antiadhésif lors du traitement d'application de la couche d'enrobage gastro-résistante (2) sur le coeur (1) de pilule.

**32.** Procédé selon l'une quelconque des revendications 21 à 31, **caractérisé en ce que** la couche contenant du carvédilol (3) est préparée en utilisant de l'hydroxypropylcellulose, de l'hydroxypropylméthylcellulose, de l'hydroxyéthylcellulose, une polyvinylpyrrolidone, un copolymère vinylpyrrolidone/acétate de vinyle, un alcool polyvinylique, un polyéthylèneglycol, un copolymère greffé alcool polyvinylique-polyéthylèneglycol ou des mélanges de ceux-ci, de préférence un mélange d'hydroxypropylcellulose et de polyéthylèneglycol, en tant qu'agent d'enrobage pelliculaire hydrosoluble.

**33.** Procédé selon la revendication 32, **caractérisé en ce que** d'autres agents auxiliaires sont utilisés.

**34.** Procédé selon l'une quelconque des revendications 21 à 33, **caractérisé en ce que** la couche contenant du carvédilol (3) est préparée par l'application subséquente de carvédilol en poudre et de l'agent liant hydrosoluble ou en pulvérisant une suspension contenant un mélange du carvédilol et le mélange aqueux de l'agent liant hydrosoluble.

**35.** Procédé selon l'une quelconque des revendications 21 à 34, **caractérisé en ce que** de l'hydroxypropylcellulose, de l'hydroxypropylméthylcellulose, de l'hydroxyéthylcellulose, de la polyvinylpyrrolidone, un copolymère vinylpyrrolidone/acétate de vinyle, un alcool polyvinylique, un polyéthylèneglycol, un copolymère greffé alcool polyvinylique-polyéthylèneglycol ou des mélanges de ceux-ci, de préférence de l'hydroxypropylcellulose, sont utilisés en tant que composés d'enrobage pelliculaire hydrosolubles pour la préparation de la couche externe (4) sur la surface de la couche contenant du carvédilol (3).

**36.** Procédé selon l'une quelconque des revendications 21 à 34, **caractérisé en ce qu'**un copolymère acide méthacrylique-acrylate d'éthyle, un copolymère acide méthacrylique-méthacrylate de méthyle, de l'acétophtalate de cellulose, du phtalate d'hydroxyméthylcellulose, de l'acétosuccinate d'hydroxypropylméthylcellulose ou de l'acétophtalate de polyvinyle, ou des mélanges de ceux-ci, de préférence un copolymère acide méthacrylique-acrylate d'éthyle, sont utilisés en tant que composé d'enrobage pelliculaire hydrosoluble pour la préparation de la couche externe (4) sur la surface de la couche contenant du carvédilol (3).

**37.** Procédé pour la préparation d'une composition pharmaceutique contenant du carvédilol sous la forme de pilules multicouches selon la revendication 21, **caractérisé en ce que**

a) 25 à 35 % en poids d'acide succinique et facultativement 25 à 35 % en poids de cellulose microcristalline sont granulés avec de l'eau contenant 0,4 à 1 % en poids de diméthylpolysiloxane par rapport au poids total des pilules, les coeurs (1) de pilules obtenus sont séchés, et

b) enrobés avec 1 à 5 % en poids de copolymère acide méthacrylique-acrylate d'éthyle, facultativement avec un mélange de 0,1 à 0,35 % en poids de propylèneglycol et de 0,1 à 0,15 % en poids d'agent antiadhésif par rapport au poids total des pilules par pulvérisation sur la surface des coeurs (1), les pilules enrobées obtenues sont facultativement séchées, puis

c) sur la surface de la couche gastro-résistante (2), une autre couche est appliquée en pulvérisant les pilules enrobées avec un mélange de 10 à 15 % en poids de carvédilol par rapport au poids et de 10 à 15 % en poids d'un mélange d'hydroxypropylméthylcellulose, de polyéthylèneglycol et facultativement d'autres agents auxiliaires ou,

la couche est préparée par l'application subséquente de 5 à 30 % en poids de carvédilol en poudre et de 5 à 30 % en poids du mélange d'hydroxyméthylcellulose, de polyéthylèneglycol et facultativement d'autres agent auxiliaires,

les pilules obtenues sont facultativement séchées, puis

d) la couche obtenue contenant du carvédilol (3) est enrobée par pulvérisation avec un mélange alcoolique de 1 à 3 % en poids de copolymère acide méthacrylique-acrylate d'éthyle et de 1 à 3 % en poids d'hydroxypropylcellulose par rapport au poids total de la pilule.

**38.** Procédé pour la préparation d'une forme posologique solide à libération lente selon l'une quelconque des revendications 1 à 14, **caractérisé en ce que** les pilules sont facultativement mélangées avec des agents auxiliaires généralement utilisés dans l'industrie pharmaceutique puis transformées en la forme galénique.

**39.** Procédé pour la préparation d'une forme posologique solide à libération lente selon la revendication 38, dans lequel 40 à 90 % en poids, de préférence 60 à 90 % en poids, de préférence encore 80 à 90 % en poids des pilules selon l'une quelconque des revendications 1 à 14, 10 à 50 % en poids, de préférence 10 à 40 % en poids, de préférence encore 10 à 20 % de la charge, facultativement 1 à 10 % en poids, de préférence 2 à 6 % en poids de l'agent désintégrant, 2 à 10 % en poids, de préférence 3 à 8 % en poids, de préférence encore 0,1 à 5 % en poids de l'agent liant et facultativement 0,1 à 1 % en poids de lubrifiant sont mélangés et compressés en comprimés.

**40.** Procédé pour la préparation d'une forme posologique solide à libération lente selon la revendication 38, dans lequel 40 à 100 % en poids, de préférence 60 à 100 % en poids, de préférence encore 80 à 100 % en poids des pilules selon l'une quelconque des revendications 1 à 14, facultativement 0,1 à 60 % en poids, de préférence 0,1 à 20 % en poids, de préférence encore 0,1 à 10 % en poids de la charge, facultativement 0,1 à 20 % en poids, de préférence 0,1 à 10 % en poids, de préférence encore 0,1 à 5 % en poids de l'agent désintégrant, 2 à 10 % en poids, de préférence 3 à 8 % en poids, de préférence encore 0,1 à 5 % en poids de l'agent liant et facultativement 0,1 à 2 % en poids, de préférence 0,1 à 1 % en poids du lubrifiant sont mélangés et introduits dans des capsules.

**41.** Procédé pour la préparation de capsules selon la revendication 40, **caractérisé en ce que** des capsules en gélatine dure sont utilisées.

**42.** Procédé pour la préparation de formes posologiques selon l'une quelconque des revendications 38 à 41, **caractérisé en ce que** les excipients généralement acceptables, de préférence le lactose, l'amidon, la cellulose en poudre, la cellulose microcristalline, le dioxyde de silicium colloïdal, un diméthylpolysiloxane ou des mélanges de ceux-ci, de toute préférence la cellulose microcristalline, le dioxyde de silicium colloïdal, un diméthylpolysiloxane ou des mélanges de ceux-ci, sont utilisés, tout agent liant pharmaceutiquement acceptable, de préférence une polyvinylpyrrolidone, l'hydroxypropylcellulose, est utilisé, tous agents désintégrants parmi les agents désintégrants pharmaceutiquement acceptés, de préférence la carboxyméthylcellulose sodique, une polyvinylpyrrolidone réticulée, l'amidon carboxyméthylé sodique, ou l'hydroxypropylcellulose peu substituée, de préférence encore la crospovidone, sont utilisés, tous lubrifiants parmi les lubrifiants pharmaceutiquement acceptables, de préférence le stéarate de calcium et de magnésium, le béhénate de glycéryle, l'acide stéarique, le talc ou les huiles végétales hydrogénées peuvent être utilisés.

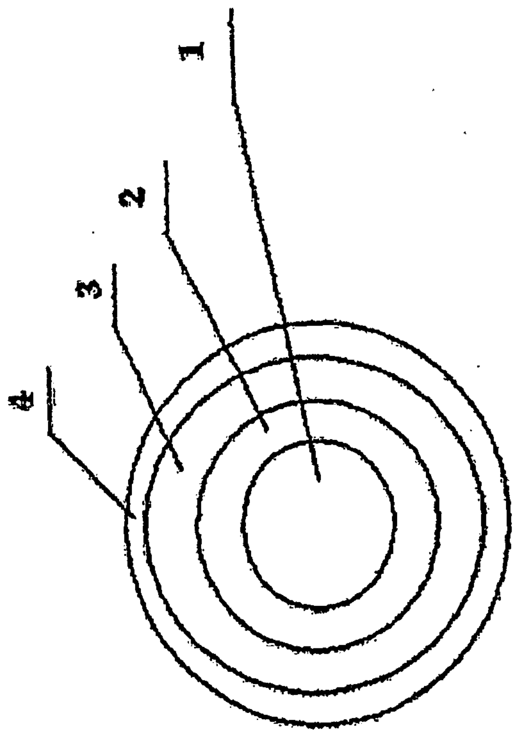


Figure 1

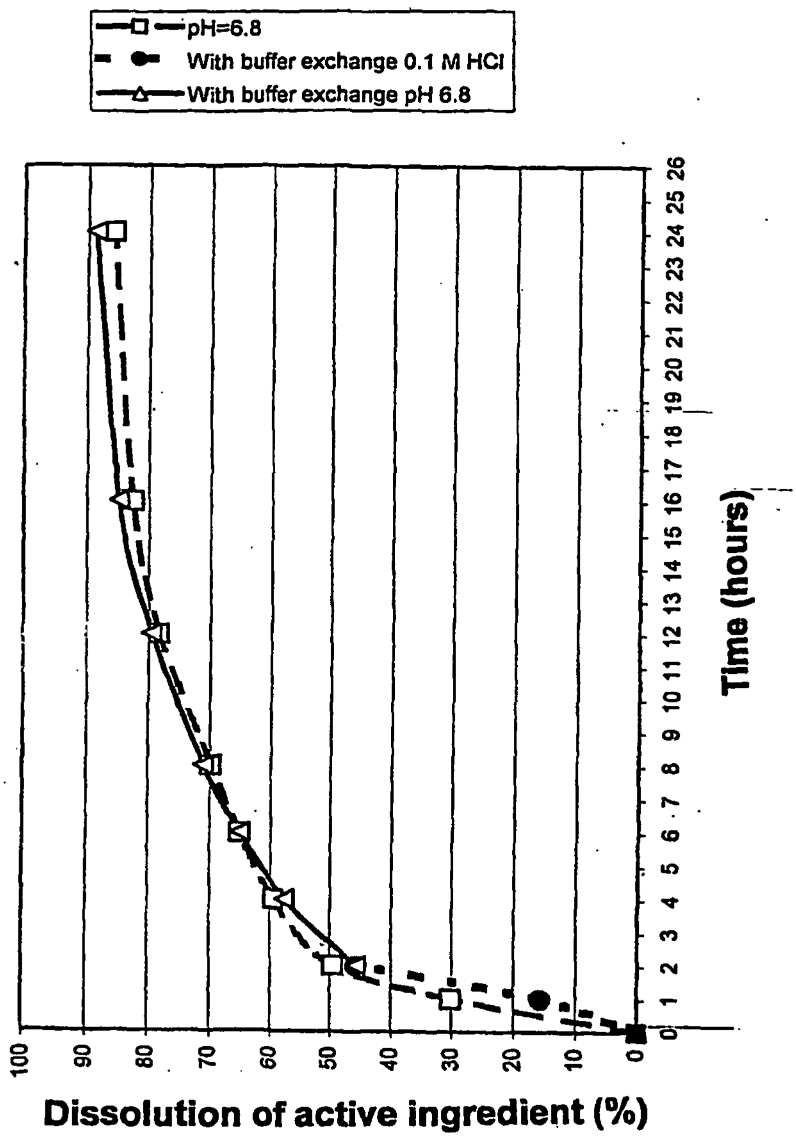
pH=6.8
With buffer exchange 0.1 M HCl
With buffer exchange pH 6.8
Time (hours)
0 1 2 3 4 5 6 7 8 9 10 11 12 13 14 15 16 17 18 19 20 21 22 23 24 25 26
Dissolution of active ingredient (%)
0 10 20 30 40 50 60 70 80 90 100

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03092622 A
- WO 2004002419 A
- WO 2004002472 A
- WO 0135958 A
- WO 03028718 A
- WO 9924017 A
- WO 02092078 A
- WO 02065834 A
- WO 2004016249 A
- WO 03024426 A
- WO 9810754 A
- WO 02080887 A
- WO 2004056336 A